# EUROPEAN PATENT APPLICATION

(11) **EP 4 501 937 A1**
(43) Date of publication of application: **05.02.2025**
(21) Application number: 23780274.9
(22) Date of filing: 24.03.2023
(51) Int. Cl.: C07F 7/18, C07D 249/14, C09J 5/02, C09J 201/00, C23C 26/00, C23F 11/00

(54) **TRIAZOLE COMPOUND, METHOD FOR SYNTHESIZING SAID TRIAZOLE COMPOUND, COUPLING AGENT AND USES THEREOF**

(30) Priority: 29.03.2022 JP 2022054330
(71) Applicant: Shikoku Chemicals Corporation, Marugame-shi, Kagawa 763-8504 (JP)
(72) Inventor: TAKASAKU Koji, Ayauta-gun, Kagawa 769-0202 (JP); YAMAJI Noriaki, Ayauta-gun, Kagawa 769-0202 (JP); KOGA Tatsuya, Ayauta-gun, Kagawa 769-0202 (JP); MURAI Takayuki, Ayauta-gun, Kagawa 769-0202 (JP); TANIOKA Miya, Ayauta-gun, Kagawa 769-0202 (JP); KATSUMURA Masato, Ayauta-gun, Kagawa 769-0202 (JP)
(74) Representative: Ipsilon
(86) International application number: PCT/JP2023/012044
(87) International publication number: WO 2023/190264

(57) **Abstract**

The present invention addresses the problem of providing: a novel triazole compound; a method for synthesizing the triazole compound; and a coupling agent. A triazole compound according to the present invention is represented by chemical formula (I). The definition of each substituent in chemical formula (I) is as described in the description.

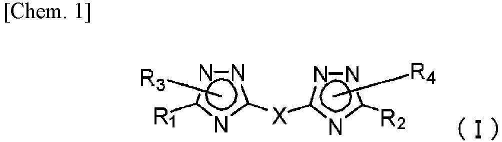

## Description

### TECHNICAL FIELD

The present invention relates to a novel triazole compound, a method for synthesizing the triazole compound, a coupling agent, and uses thereof.

### BACKGROUND ART

In the related art, a coupling agent has been used to bond materials having properties different from each other, such as an organic material and an inorganic material, and the coupling agent has been used in the fields of electronic materials, coating materials, primers, adhesives, and the like. Therefore, the coupling agent is an essential chemical agent for the development and production of the composite material.

For example, there is known a coupling agent (silane coupling agent) using a compound composed of an organic substance and silicon, and this silane coupling agent functions as an intermediary for binding an organic material and an inorganic material, which are not usually compatible with each other.

In addition, the triazole compound has a function of preventing metal corrosion and a function of curing an epoxy resin and a urethane resin, and thus various coupling agents using a triazole compound have been proposed.

As a triazole compound that can be used for the coupling agent, for example, Patent Literature 1 proposes a triazole silane compound in which an alkoxysilyl group is introduced into a specific triazole ring. In addition, Patent Literature 2 proposes a triazole silane compound having specific two triazole rings and a disulfide bond (-S-S-), in which -CO-NH-(CH₂)ₘ-Si(OR)₃ is introduced into the triazole ring.

### CITATION LIST

### PATENT LITERATURE

Patent Literature 1: WO2018/186476
Patent Literature 2: WO2015/002158

### SUMMARY OF INVENTION

### TECHNICAL PROBLEM

In recent years, in the fields of electrical and electronic materials, there has been a demand for enhancing adhesiveness between materials in order to cope with miniaturization, thinning, high precision, high-speed propagation, and the like. The triazole compound as disclosed in Patent Literature 1 and Patent Literature 2 can also exhibit excellent adhesiveness; however, there is a demand for a compound that is capable of exhibiting further higher adhesiveness.

The present invention has been made in consideration of the above-described problems, and an object of the present invention is to provide a novel triazole compound, a synthesis method therefor, and a coupling agent.

### SOLUTION TO PROBLEM

As a result of intensive studies to achieve the above-described object, the inventors of the present invention have found that the above-described object can be achieved by linking two triazole rings in a triazole compound having two triazole rings, to each other by a specific linking group, whereby the present invention has been completed.

That is, the present invention is as described in [1] to [20] below.
[1] A triazole compound represented by Chemical Formula (I). 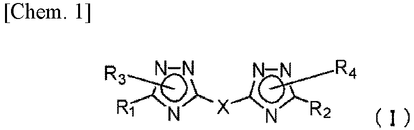 (In Formula (I),
   R₁ and R₂, which are the same or different from each other, represent a hydrogen atom, a linear or branched alkyl group having 1 to 6 carbon atoms, an aryl group, an aralkyl group, an amino group, a hydroxyl group, or an alkylthio group having 1 to 6 carbon atoms,
   R₃ and R₄, which are the same or different from each other, represent a hydrogen atom or a group represented by -(CH₂)ₘ-Si(OR)₃, where R represents a methyl group or an ethyl group and m represents an integer of 1 to 18,
   provided that R₃ and R₄ are not hydrogen atoms at the same time, and
   X represents a phenylene group or a group represented by -NH- or -(CH₂)ₙ-, where n represents an integer of 0 to 12.)
[2] A method for synthesizing the triazole compound according to [1], which includes reacting a triazole compound represented by Chemical Formula (II) with a compound represented by Chemical Formula (III). 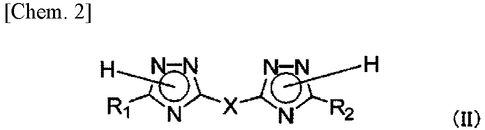 (In Formula (II), R₁, R₂, and X are the same as those described above.) 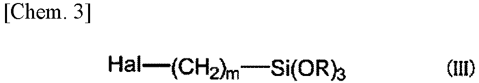 (In Formula (III), Rand m are the same as those described above, and Hal represents a chlorine atom, a bromine atom, or an iodine atom.)
[3] A coupling agent containing a triazole compound represented by Chemical Formula (IV) as a component. 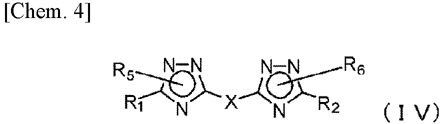 (In Formula (IV),
   R₁ and R₂, which are the same or different from each other, represent a hydrogen atom, a linear or branched alkyl group having 1 to 6 carbon atoms, an aryl group, an aralkyl group, an amino group, a hydroxyl group, or an alkylthio group having 1 to 6 carbon atoms,
   R₅ and R₆, which are the same or different from each other, represent a hydrogen atom or a group represented by -(CH₂)ₘ-Si(OR)₃₋ₚ(OH)ₚ, where R represents a methyl group or an ethyl group, m represents an integer of 1 to 18, and p represents an integer of 0 to 3, and
   X represents a phenylene group or a group represented by -NH- or -(CH₂)ₙ-, where n represents an integer of 0 to 12.)
[4] A surface treatment liquid containing a triazole compound represented by Chemical Formula (IV). 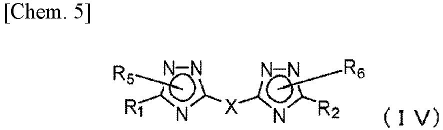 (In Formula (IV),
   R₁ and R₂, which are the same or different from each other, represent a hydrogen atom, a linear or branched alkyl group having 1 to 6 carbon atoms, an aryl group, an aralkyl group, an amino group, a hydroxyl group, or an alkylthio group having 1 to 6 carbon atoms,
   R₅ and R₆, which are the same or different from each other, represent a hydrogen atom or a group represented by -(CH₂)ₘ-Si(OR)₃₋ₚ(OH)ₚ, where R represents a methyl group or an ethyl group, m represents an integer of 1 to 18, and p represents an integer of 0 to 3, and
   X represents a phenylene group or a group represented by -NH- or -(CH₂)ₙ-, where n represents an integer of 0 to 12.)
[5] The surface treatment liquid according to [4], which is used for treating a surface of at least one selected from the group consisting of a metal, an inorganic material, and a resin material.
[6] The surface treatment liquid according to [4], which is used for adhering, to each other, two materials selected from the group consisting of a metal, an inorganic material, and a resin material.
[7] The surface treatment liquid according to [5] or [6], in which the metal is at least one selected from the group consisting of copper, aluminum, titanium, nickel, tin, iron, silver, gold, and an alloy of these metals.
[8] A surface treatment method, which includes bringing the surface treatment liquid according to [4] into contact with a surface of at least one selected from the group consisting of a metal, an inorganic material, and a resin material.
[9] The surface treatment method according to [8], in which the metal is at least one selected from the group consisting of copper, aluminum, titanium, nickel, tin, iron, silver, gold, and an alloy of these metals.
[10] The surface treatment method according to [9], in which the metal is copper or a copper alloy.
[11] The surface treatment method according to [10], in which before the surface treatment liquid is brought into contact with a surface of copper or a copper alloy, an aqueous solution containing copper ions is brought into contact with the surface of the copper or the copper alloy.
[12] The surface treatment method according to [10] or [11], in which after the surface treatment liquid is brought into contact with a surface of copper or a copper alloy, an acidic aqueous solution or an alkaline aqueous solution is brought into contact with the surface of the copper or the copper alloy.
[13] An adhesion method, which includes bringing the surface treatment liquid according to [4] into contact with at least one selected from the group consisting of a metal, an inorganic material, and a resin material to form a chemical conversion coating on the at least one, and carrying out mutual adhesion through the chemical conversion coating.
[14] An adhesion method for a metal to a resin material, including bringing the surface treatment liquid according to [4] into contact with at least one of a metal or a resin material to form a chemical conversion coating on the at least one, and adhering the metal and the resin material to each other through the chemical conversion coating.
[15] A printed wiring board, in which two materials selected from the group consisting of a metal, an inorganic material, and a resin material are adhered to each other through a chemical conversion coating formed from the surface treatment liquid according to [4].
[16] A semiconductor wafer in which two materials selected from the group consisting of a metal, an inorganic material, and a resin material are adhered to each other through a chemical conversion coating formed from the surface treatment liquid according to [4].
[17] An insulating composition which contains the coupling agent according to [3] and a resin material or an inorganic material.
[18] An insulating material which contains the insulating composition according to [17].
[19] A printed wiring board which includes an insulating layer obtained from the insulating composition according to [17].
[20] A semiconductor wafer which includes an insulating layer obtained from the insulating composition according to [17].

### ADVANTAGEOUS EFFECTS OF INVENTION

The triazole compound according to the present invention is a compound in which two triazole rings are bonded to each other, and thus interaction with a metal, a resin material, or the like can be enhanced. In addition, since the two triazole rings are bonded to each other through a linking group selected from the group consisting of a phenylene group, a group represented by -NH-, and a group represented by - (CH₂)ₙ-, the triazole compound is less likely to be decomposed by heat or an alkali.

In addition, since the coupling agent according to the present invention contains a triazole compound in which two triazole rings are linked by a linking group, it is possible to enhance the adhesiveness between materials having properties different from each other. According to the surface treatment liquid containing this triazole compound, it is possible to enhance the adhesiveness between two materials having qualities different from each other, that is, between a metal and an inorganic material, between a metal and a resin material, and between an inorganic material and a resin material.

### DESCRIPTION OF EMBODIMENTS

Hereinafter, the present invention will be described in detail. It is noted that the present invention is not limited to the embodiments described below.

### (Triazole compound)

A triazole compound according to the present invention is represented by Chemical Formula (I) (hereinafter, may be referred to as a triazole compound (I)). 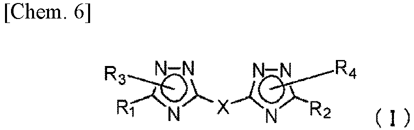 (In Formula (I),
R₁ and R₂, which are the same or different from each other, represent a hydrogen atom, a linear or branched alkyl group having 1 to 6 carbon atoms, an aryl group, an aralkyl group, an amino group, a hydroxyl group, or an alkylthio group having 1 to 6 carbon atoms,
R₃ and R₄, which are the same or different from each other, represent a hydrogen atom or a group represented by -(CH₂)ₘ-Si(OR)₃, where R represents a methyl group or an ethyl group and m represents an integer of 1 to 18,
provided that R₃ and R₄ are not hydrogen atoms at the same time, and
X represents a phenylene group or a group represented by -NH- or -(CH₂)ₙ-, where n represents an integer of 0 to 12.)

In Formula (I), R₁ and R₂ represent a hydrogen atom, a linear or branched alkyl group having 1 to 6 carbon atoms, an aryl group, an aralkyl group, an amino group, a hydroxyl group, or an alkylthio group having 1 to 6 carbon atoms. R₁ and R₂ may be the same or different from each other.

Specific examples of the linear alkyl group having 1 to 6 carbon atoms include a methyl group, an ethyl group, an n-propyl group, an n-butyl group, an n-pentyl group, and an n-hexyl group. The branched alkyl group having 1 to 6 carbon atoms is a branched alkyl group having 3 to 6 carbon atoms. Specific examples thereof include an isopropyl group, an isobutyl group, an s-butyl group, a t-butyl group, an isopentyl group, an s-pentyl group, a t-pentyl group, an isohexyl group, an s-hexyl group, and a t-hexyl group. Specific examples of the aryl group include a phenyl group, an o-tolyl group, an m-tolyl group, a p-tolyl group, a xylyl group, a trimethylphenyl group, a tetramethylphenyl group, a 1-naphthyl group, and a 2-naphthyl group. Specific examples of the aralkyl group include a benzyl group, a phenylethyl group, a phenylpropyl group, and a methylbenzyl group. Specific examples of the alkylthio group having 1 to 6 carbon atoms include a methylthio group, an ethylthio group, a propylthio group, an isopropylthio group, a butylthio group, an isobutylthio group, a sec-butylthio group, a tert-butylthio group, a pentylthio group, and a hexylthio group.

It is more preferable that R₁ and R₂, which are the same or different from each other, are a hydrogen atom, an amino group, or an alkylthio group having 1 to 6 carbon atoms.

R₃ and R₄ are a hydrogen atom or a group represented by -(CH₂)ₘ-Si(OR)₃ (R represents a methyl group or an ethyl group, and m represents an integer of 1 to 18). R₃ and R₄ may be the same or different from each other; however, R₃ and R₄ are not hydrogen atoms at the same time.

In the group represented by -(CH₂)ₘ-Si(OR)₃, it is preferable that m is an integer of 2 to 12, it is more preferable that m is an integer of 3 to 10, and it is still more preferable that m is an integer of 3 to 8.

X represents a phenylene group or a group represented by -NH- or -(CH₂)ₙ- (n is an integer of 0 to 12).

X is preferably a group represented by -(CH₂)ₙ-. In addition, it is more preferable that n is an integer of 0 to 10, and it is still more preferable that n is an integer of 0 to 8, which is particularly preferably 0 to 6.

In the compound represented by Chemical Formula (I) (triazole compound (I)), it is preferable that R₃ and R₄ are bonded to an N atom at the 1-position or the 2-position of the triazole ring.

Examples of the triazole compound (I) include triazole compounds represented by Chemical Formula (I-1) to Chemical Formula (I-8).

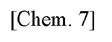

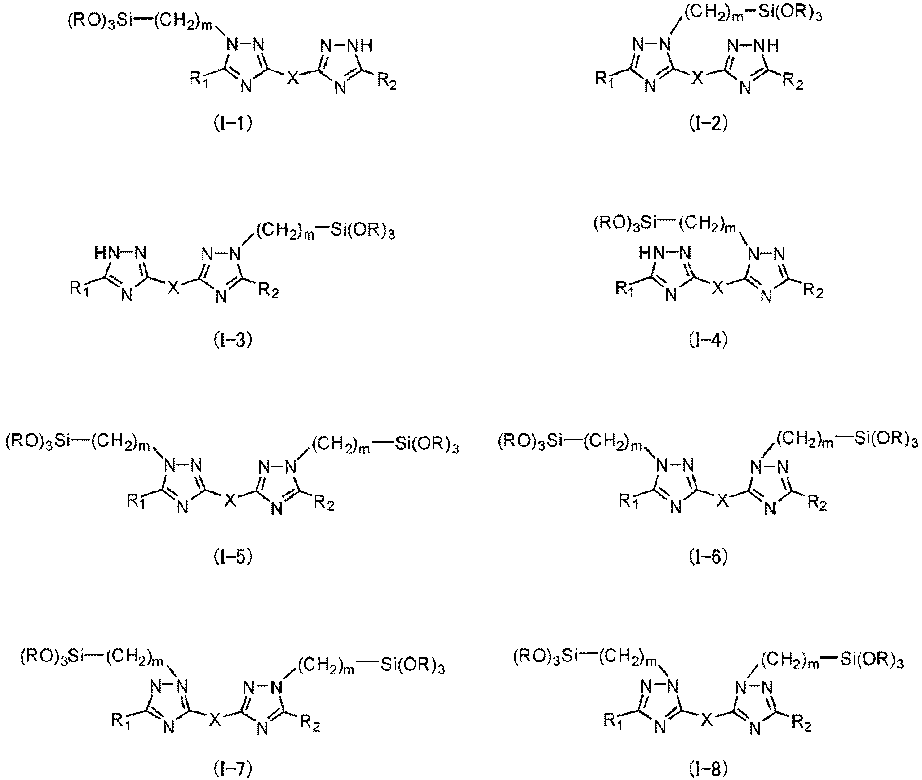

(In Formulae (I-1) to (I-8), R₁, R₂, m, R, and X are the same as those described above.)

Specific examples of the triazole compound (I) where R₃ is hydrogen and R₄ is bonded to N at the 1-position of the triazole ring (the compound represented by Formula (I-3)), include:
1-(trimethoxysilyl)methyl-3,3'-(1,4-phenylene)bis(1,2,4-triazole),
1-[2-(triethoxysilyl)ethyl]-3,3'-(1,2-phenylene)bis(5-methyl-1,2,4-triazole),
1-[3 -(triethoxysilyl)propyl]-3,3 '-(1,3 -phenylene)bis(5 -butyl-1 ,2,4-triazole),
1-[3-(triethoxysilyl)propyl]-3,3'-(1,4-phenylene)bis(5-amino-1,2,4-triazole),
1-[6-(triethoxysilyl)hexyl]-3,3'-(1,4-phenylene)bis(5-ethylthio-1,2,4-triazole),
1-[3-(triethoxysilyl)propyl]-3,3'-iminobis(5-ethyl-1,2,4-triazole),
1-[3-(triethoxysilyl)propyl]-3,3'-iminobis(5-p-tolyl-1,2,4-triazole),
1-[3-(triethoxysilyl)propyl]-3,3'-iminobis(5-atnino-1,2,4-triazole),
1-[8-(trimethoxysilyl)octyl]-3,3'-iminobis(5-isopropyl-1,2,4-triazole),
1-[3-(triethoxysilyl)propyl]-3,3'-bis(5-methyl-1,2,4-triazole),
1-[3-(triethoxysilyl)propyl]-3,3'-bis(5-pentyl-1,2,4-triazole),
1-[3-(triethoxysilyl)propyl]-3,3'-bis(5-amino-1,2,4-triazole),
1-[10-(triethoxysilyl)decyl] -3,3'-bis(5 -phenyl-1 ,2,4-triazole),
1-[3-(triethoxysilyl)propyl]-5-amino-5'-methyl-3,3'-bi-1,2,4-triazole,
1-[3-(triethoxysilyl)propyl]-3,3 '-methylene bis(5-isobutyl-1,2,4-triazole),
1-[3-(triethoxysilyl)propyl]-3,3 '-methylene bis(5-hydroxy-1,2,4-triazole),
1-[3-(triethoxysilyl)propyl]-3,3'-methylene bis(5-amino-1,2,4-triazole),
1-[6-(trimethoxysilyl)hexyl]-3,3'-methylene bis(5-amino-1,2,4-triazole),
1-[3-(triethoxysilyl)propyl]-3,3'-ethylene bis(5-ethyl-1,2,4-triazole),
1-[3-(triethoxysilyl)propyl]-3,3'-ethylene bis(5-amino-1,2,4-triazole),
1-[12-(triethoxysilyl)dodecyl]-3,3'-ethylene bis(5-amino-1,2,4-triazole),
1 -[3 -(triethoxysilyl)propyl]-3,3 '-tetramethylene bis(5-benzyl-1,2,4-triazole),
1-[3-(triethoxysilyl)propyl]-3,3'-tetramethylene bis(5-butylthio-1,2,4-triazole),
1 -[3 -(triethoxysilyl)propyl]-3,3 '-tetramethylene bis(5-amino-1,2,4-triazole),
1-[3-(triethoxysilyl)propyl]-3,3'-pentamethylene bis(1,2,4-triazole),
1-[3-(triethoxysilyl)propyl]-3,3'-pentamethylene bis(5-amino-1,2,4-triazole),
1-[4-(triethoxysilyl)butyl]-3,3'-pentatnethylene bis(5-amino-1,2,4-triazole),
1 -[3 -(triethoxysilyl)propyl]-3,3 '-hexamethylene bis(5-amino-1,2,4-triazole),
1-[3-(triethoxysilyl)propyl]-3,3'-octamethylene bis(5-amino-1,2,4-triazole),
1-[2-(trimethoxysilyl)ethyl]-3,3'-nonamethylene bis(5-amino-1,2,4-triazole),
1-[2-(trimethoxysilyl)ethyl]-3,3'-decamethylene bis(5-amino-1,2,4-triazole),
1-[3-(triethoxysilyl)propyl]-3,3'-undecamethylene bis(5-amino-1,2,4-triazole), and
1-[3-(triethoxysilyl)propyl]-3,3'-dodecamethylene bis(5-amino-1,2,4-triazole).

Specific examples of the triazole compound (I) where R₃ and R₄, which are the same or different from each other, are a group represented by -(CH₂)ₘ-Si(OR)₃, and R₃ and R₄ are bonded to N at the 1-position of the triazole ring(the compound represented by Formula (I-5)), include:
3,3'-(1,4-phenylene)bis[1-(trimethoxysilyl)methyl-1,2,4-triazole],
3,3'-(1,2-phenylene)bis {1-[2-(triethoxysilyl)ethyl]-5-methyl-1,2,4-triazole},
3,3'-(1,3 -phenylene)bis {1-[3-(triethoxysilyl)propyl]-5-butyl-1,2,4-triazole},
3,3'-(1,4-phenylene)bis{1-[3-(triethoxysilyl)propyl]-5-amino-1,2,4-triazole},
3,3'-(1,4-phenylene)bis {1-[6-(triethoxysilyl)hexyl]-5-ethylthio-1,2,4-triazole},
3,3 '-iminobis {1-[3-(triethoxysilyl)propyl]-5-ethyl-1,2,4-triazole},
3,3 '-iminobis {1-[3-(triethoxysilyl)propyl]-5-p-tolyl-1,2,4-triazole},
3,3'-iminobis{1-[3-(triethoxysilyl)propyl]-5-amino-1,2,4-triazole},
3,3 '-iminobis {1-[8-(trimethoxysilyl)octyl]-5-isopropyl-1,2,4-triazole},
3,3 '-bis { 1-[3-(triethoxysilyl)propyl]-5-pentyl-1,2,4-triazole},
3,3'-bis {1-[3-(triethoxysilyl)propyl]-5-amino-1,2,4-triazole},
3,3'-bis {1-[10-(triethoxysilyl)decyl]-5 -phenyl-1,2,4-triazole},
1,1'-bis [3-(triethoxysilyl)propyl]-5-amino-5'-methyl-3,3'-bi-1,2,4-triazole,
3,3 '-methylene bis{1-[3-(triethoxysilyl)propyl]-5-isobutyl-1,2,4-triazole},
3,3 '-methylene bis {1-[3-(triethoxysilyl)propyl]-5-hydroxy-1,2,4-triazole},
3,3'-methylene bis{1-[3-(triethoxysilyl)propyl]-5-amino-1,2,4-triazole},
3,3'-methylene bis{1-[6-(trimethoxysilyl)hexyl]-5-amino-1,2,4-triazole},
3,3'-ethylene bis{1-[3-(triethoxysilyl)propyl]-5-ethyl-1,2,4-triazole},
3,3'-ethylene bis{1-[3-(triethoxysilyl)propyl]-5-amino-1,2,4-triazole},
3,3'-ethylene bis{1-[12-(triethoxysilyl)dodecyl]-5-amino-1,2,4-triazole},
3,3'-tetramethylene bis{1-[3-(triethoxysilyl)propyl]-5-benzyl-1,2,4-triazole},
3,3 '-tetramethylene bis{1-[3-(triethoxysilyl)propyl]-5-butylthio-1,2,4-triazole},
3,3 '-tetramethylene bis{1-[3-(triethoxysilyl)propyl]-5-amino-1,2,4-triazole},
3,3'-pentamethylene bis{1-[3 -(triethoxy silyl)propyl]-1,2,4-triazole},
3,3 '-pentamethylene bis {1-[3-(triethoxysilyl)propyl]-5-amino-1,2,4-triazole},
3,3'-pentamethylene bis{1-[4-(triethoxysilyl)butyl] -5 -amino-1,2,4-triazole},
3,3 '-hexamethylene bis {1-[3-(triethoxysilyl)propyl]-5-amino-1,2,4-triazole},
3,3 '-octamethylene bis{1-[3-(triethoxysilyl)propyl]-5-amino-1,2,4-triazole},
3,3'-nonamethylene bis{1-[2-(trimethoxysilyl)ethyl]-5-amino-1,2,4-triazole},
3,3'-decamethylene bis{1-[2-(trimethoxysilyl)ethyl]-5-amino-1,2,4-triazole},
3,3'-undecamethylene bis{1-[3-(triethoxysilyl)propyl]-5-amino-1,2,4-triazole}, and
3,3'-dodecamethylene bis{1-[3-(triethoxysilyl)propyl]-5-amino-1,2,4-triazole}.

The triazole compound (I) can be obtained by reacting a triazole compound represented by Chemical Formula (II) (hereinafter, may be referred to as a triazole compound (II)) with a halogenated alkyl silane compound represented by Chemical Formula (III) (hereinafter, may be referred to as a halogenated alkyl silane compound (III)).

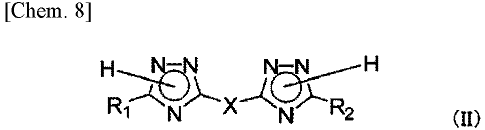

(In Formula (II), R₁, R₂, and X are the same as those described above.)

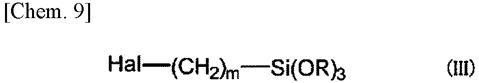

(In Formula (III), Rand m are the same as those described above. Hal represents a chlorine atom, a bromine atom, or an iodine atom.)

In Formula (II), R₁, R₂, and X are the same as those indicated in Formula (I), and the same is applied to the preferred examples thereof.

In Formula (III), R and m are the same as those indicated in Formula (I), and the same is applied to the preferred examples thereof.

Hal in Formula (III) is a chlorine atom, a bromine atom, or an iodine atom, and a chlorine atom or a bromine atom is preferable from the viewpoint of reactivity and ease of obtaining a raw material.

Examples of the triazole compound (II) include:
3,3'-(1,4-phenylene)bis(1,2,4-triazole),
3,3'-(1,4-phenylene)bis(5-methyl-1,2,4-triazole),
3,3'-(1,2-phenylene)bis(5-ethyl-1,2,4-triazole),
3,3'-(1,4-phenylene)bis(5-isopropyl-1,2,4-triazole),
3,3'-(1,3-phenylene)bis(5 -butyl-1,2,4-triazole),
3,3'-(1,4-phenylene)bis(5-phenyl-1,2,4-triazole),
3,3'-(1,4-phenylene)bis(5-amino-1,2,4-triazole),
3,3'-(1,4-phenylene)bis(5-hydroxy-1,2,4-triazole),
3,3'-(1,4-phenylene)bis(5-ethylthio-1,2,4-triazole),
5-amino-3,3'-(1,4-phenylene)bis(1,2,4-triazole),
3,3'-iminobis(1,2,4-triazole),
3,3'-iminobis(5-methyl-1,2,4-triazole),
3,3'-iminobis(5 -hexyl-1,2,4-triazole),
3,3'-iminobis(5 -phenyl-1,2,4-triazole),
3,3'-iminobis(5 -benzyl-1,2,4-triazole),
3,3'-iminobis(5 -amino-1,2,4-triazole),
3,3'-iminobis(5 -hydroxy-1,2,4-triazole),
3,3'-iminobis(5-methylthio-1,2,4-triazole),
5-amino-3,3 '-iminobis(1,2,4-triazole),
3,3'-bi-1,2,4-triazole,
3,3'-bis(5-methyl-1,2,4-triazole),
3,3'-bis(5-ethyl-1,2,4-triazole),
3,3'-bis(5-pentyl-1,2,4-triazole),
3,3'-bis(5-p-tolyl-1,2,4-triazole),
3,3'-bis(5-benzyl-1,2,4-triazole),
3,3'-bis(5-amino-1,2,4-triazole),
3,3'-bis(5-hydroxy-1,2,4-triazole),
3,3'-bis(5-hexylthio-1,2,4-triazole),
5-amino-5'-methyl-3,3'-bi-1,2,4-triazole,
3,3'-methylene bis(1,2,4-triazole),
3,3'-methylene bis(5-methyl-1,2,4-triazole),
3,3 '-methylene bis(5-isobutyl-1,2,4-triazole),
3,3'-methylene bis(5-phenyl-1,2,4-triazole),
3,3'-methylene bis(5-phenethyl-1,2,4-triazole),
3,3'-methylene bis(5-amino-1,2,4-triazole),
3,3'-methylene bis(5-hydroxy-1,2,4-triazole),
3,3'-methylene bis(5-methylthio-1,2,4-triazole),
5-amino-5'-ethyl-3,3'-methylene bis(1,2,4-triazole),
3,3'-ethylene bis(1,2,4-triazole),
3,3'-ethylene bis(5-propyl-1,2,4-triazole),
3,3'-ethylene bis(5-hexyl-1,2,4-triazole),
3,3'-ethylene bis(5-m-tolyl-1,2,4-triazole),
3,3'-ethylene bis(5-amino-1,2,4-triazole),
3,3'-ethylene bis(5-hydroxy-1,2,4-triazole),
3,3'-ethylene bis(5-isopropylthio-1,2,4-triazole),
5-amino-3,3'-ethylene bis(1,2,4-triazole),
3,3'-trimethylene bis(1,2,4-triazole),
5-methyl-3,3'-trimethylene bis(1,2,4-triazole),
3,3'-trimethylene bis(5-butyl-1,2,4-triazole),
3,3'-trimethylene bis(5-phenyl-1,2,4-triazole),
3,3'-trimethylene bis(5-amino-1,2,4-triazole),
3,3'-trimethylene bis(5-propylthio-1,2,4-triazole),
5-amino-5'-benzyl-3,3'-trimethylene bis(1,2,4-triazole),
3,3'-tetramethylene bis(1,2,4-triazole),
3,3'-tetramethylene bis(5-ethyl-1,2,4-triazole),
3,3'-tetramethylene bis(5-hexyl-1,2,4-triazole),
3,3'-tetramethylene bis(5-benzyl-1,2,4-triazole),
3,3'-tetramethylene bis(5-amino-1,2,4-triazole),
3,3'-tetramethylene bis(5 -hydroxy-1,2,4-triazole),
3,3'-tetramethylene bis(5-butylthio-1,2,4-triazole),
5-amino-3,3'-tetramethylene bis(1,2,4-triazole),
3,3'-pentamethylene bis(1,2,4-triazole),
3,3'-pentamethylene bis(5-propyl-1,2,4-triazole),
3,3'-pentamethylene bis(5-amino-1,2,4-triazole),
3,3'-pentamethylene bis(5-hydroxy-1,2,4-triazole),
3,3'-pentamethylene bis(5-pentylthio-1,2,4-triazole),
5-amino-5'-phenyl-3,3'-pentamethylene bis(1,2,4-triazole),
3,3'-hexamethylene bis(1,2,4-triazole),
3,3'-hexamethylene bis(5-pentyl-1,2,4-triazole),
3,3'-hexamethylene bis(5-amino-1,2,4-triazole),
5-hydroxy-3,3'-hexamethylene bis(1,2,4-triazole),
5-phenyl-3,3'-hexamethylene bis(1,2,4-triazole),
5-amino-5'-methylthio-3,3'-hexamethylene bis(1,2,4-triazole),
3,3'-heptamethylene bis(1,2,4-triazole),
3,3'-heptamethylene bis(5-tert-butyl-1,2,4-triazole),
3,3'-heptamethylene bis(5-phenyl-1,2,4-triazole),
3,3'-heptamethylene bis(5-amino-1,2,4-triazole),
5-amino-5'-hydroxy-3,3'-heptamethylene bis(1,2,4-triazole),
3,3'-octamethylene bis(1,2,4-triazole),
3,3'-octamethylenebis(5-isobutyl-1,2,4-triazole),
3,3'-octamethylene bis(5-amino-1,2,4-triazole),
3,3'-octamethylene bis(5-hydroxy-1,2,4-triazole),
5-propylthio-3,3'-octamethylene bis(1,2,4-triazole),
3,3'-nonamethylene bis(1,2,4-triazole),
3,3 '-nonamethylene bis(5-isopentyl-1,2,4-triazole),
5-phenyl-3,3'-nonamethylene bis(1,2,4-triazole),
3,3'-nonamethylene bis(5 -amino-1,2,4-triazole),
3,3'-nonamethylene bis(5-methylthio-1,2,4-triazole),
3,3'-decamethylene bis(1,2,4-triazole),
3,3'-decamethylene bis(5-methyl-1,2,4-triazole),
3,3 '-decamethylene bis(5-amino-1,2,4-triazole),
5-ethyl-3,3'-decamethylene bis(1,2,4-triazole),
3,3'-undecamethylene bis(1,2,4-triazole),
3,3 '-undecamethylene bis(5-propyl-1,2,4-triazole),
3,3'-undecamethylenebis(5-amino-1,2,4-triazole),
3,3'-undecamethylene bis(5-methylthio-1,2,4-triazole),
3,3'-dodecamethylene bis(1,2,4-triazole),
3,3 '-dodecamethylene bis(5-ethyl-1,2,4-triazole),
3,3'-dodecamethylene bis(5-amino-1,2,4-triazole), and
3,3'-undecamethylene bis(5-hydroxy-1,2,4-triazole).

The triazole compound (II) exemplified above can be synthesized, for example, in accordance with the synthesis method described in Chem. Eur. J., 18, 16742 (2012), J. Applied. Chem., 82, 276 (2009), or the specification of United States Patent No. 2744116.

Examples of the halogenated alkyl silane compound (III) include:
chloromethyltrimethoxysilane,
chloromethyltriethoxysilane,
2-chloroethyltrimethoxysilane,
2-chloroethyltriethoxysilane,
3-chloropropyltrimethoxysilane,
3-chloropropyltriethoxysilane,
3-bromopropyltrimethoxysilane,
3-bromopropyltriethoxysilane,
3-iodopropyltrimethoxysilane,
3-iodopropyltriethoxysilane,
4-bromobutyltrimethoxysilane,
4-bromobutyltriethoxysilane,
5 -bromopentyltrimethoxysilane,
5 -bromopentyltriethoxysilane,
6-bromohexyltrimethoxysilane,
6-bromohexyltriethoxysilane,
8-bromooctyltrimethoxysilane,
8-bromooctyltriethoxysilane,
10-bromodecyltrimethoxysilane,
10-bromodecyltriethoxysilane,
12-bromododecyltrimethoxysilane, and
12-bromododecyltriethoxysilane.

The triazole compound (I) according to the present invention can generally be synthesized in a high yield, for example, by reacting the triazole compound (II) with the halogenated alkyl silane compound (III) in an appropriate amount of a reaction solvent at an appropriate reaction temperature for an appropriate reaction time in the presence of a dehydrohalogenating agent. In the following reaction scheme (A), a reaction scheme for obtaining a triazole compound (I) in which R₃ is a hydrogen atom and R₄ is a group represented by -(CH₂)ₘ-Si(OR)₃ is shown.

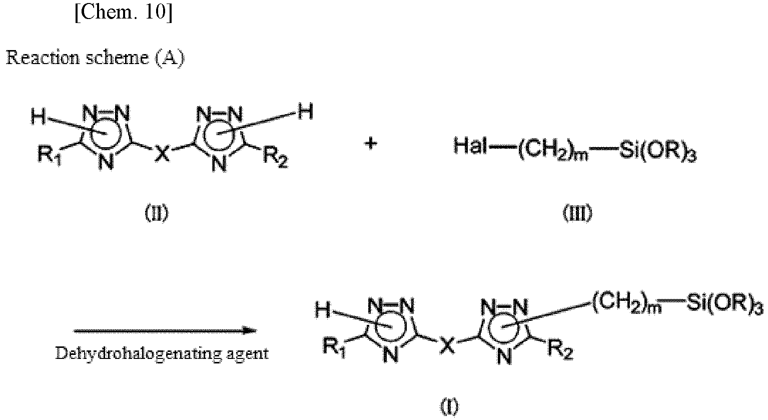

(In the formulae, R₁, R₂, X, R, Hal, and m are the same as those described above.)

The reaction solvent is not particularly limited as long as it is an inert solvent with respect to the triazole compound (II) and the halogenated alkyl silane compound (III). Examples thereof include:
hydrocarbon-based solvents such as hexane, toluene, and xylene;
ether-based solvents such as diethyl ether, tetrahydrofuran, dioxane, and cyclopentyl methyl ether;
ester-based solvents such as ethyl acetate and butyl acetate;
alcohol-based solvents such as methanol and ethanol;
amide-based solvents such as N, N-dimethylformamide, N, N-dimethylacetamide, and N-methylpyrrolidone;
ketone-based solvents such as acetone, methyl ethyl ketone, methyl isobutyl ketone, and cyclohexanone; and
acetonitrile, dimethyl sulfoxide, and hexamethyl phosphoramide.

Examples of the dehydrohalogenating agent include:
alkali metal alkoxides such as sodium methoxide, sodium ethoxide, potassium methoxide, and potassium t-butoxide;
alkali metal carbonates such as sodium carbonate and potassium carbonate; and
organic bases such as triethylamine and diazabicycloundecene, and sodium hydride.

The reaction of the triazole compound (II) with the halogenated alkyl silane compound (III) proceeds stoichiometrically as shown in the above-described reaction scheme (A). However, in consideration of factors such as the reaction temperature and the reaction time, as well as the kinds of raw materials and reaction solvents to be used, and the reaction scale, the use amount (charge amount) of the halogenated alkyl silane compound (III) to the use amount (charge amount) of the triazole compound (II) is preferably such that 0.8 to 1.2 equivalents of the halogenated alkylsilane compound (III) are used with respect to each -NH of the triazole ring, which serves as a reaction point.

In the case where the charge amount of the halogenated alkyl silane compound (III) per reaction point (-NH) is more than 1.2 equivalents, there is a concern that the compound is polymerized to be gelated, and in the case where the charge amount thereof is less than 0.8 equivalents, there is such a concern that the purity of the product decreases or the separation operation of the product is complicated.

In addition, since the dehydrohalogenating agent is used to neutralize hydrogen halide that is generated as a by-product by the reaction between the triazole compound (II) and the halogenated alkyl silane compound (III), the use amount (charge amount) of the dehydrohalogenating agent may be any amount that is equal to or more than the use amount of the halogenated alkyl silane compound (III).

The reaction temperature is not particularly limited as long as it is in a temperature range in which the -NH of the triazole ring of the triazole compound (II) reacts with the halogenated alkyl silane compound (III); however, the reaction temperature is preferably in a range of 0°C to 150°C and more preferably in a range of 5°C to 100°C.

The reaction time is appropriately determined according to the set reaction temperature; however, the reaction time is preferably in a range of 30 minutes to 24 hours and more preferably in a range of 1 to 8 hours.

### (Coupling agent)

A coupling agent according to the present invention contains, as a component, a triazole compound represented by Chemical Formula (IV) (hereinafter, may be referred to as a triazole compound (IV)). 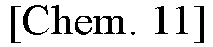 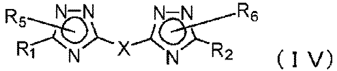 (In Formula (IV),
R₁ and R₂, which are the same or different from each other, represent a hydrogen atom, a linear or branched alkyl group having 1 to 6 carbon atoms, an aryl group, an aralkyl group, an amino group, a hydroxyl group, or an alkylthio group having 1 to 6 carbon atoms,
R₅ and R₆, which are the same or different from each other, represent a hydrogen atom or a group represented by -(CH₂)ₘ-Si(OR)₃₋ₚ(OH)ₚ, where R represents a methyl group or an ethyl group, m represents an integer of 1 to 18, and p represents an integer of 0 to 3, and
X represents a phenylene group or a group represented by -NH- or -(CH₂)ₙ-, where n represents an integer of 0 to 12.)

In Formula (IV), R₁ and R₂ represent a hydrogen atom, a linear or branched alkyl group having 1 to 6 carbon atoms, an aryl group, an aralkyl group, an amino group, a hydroxyl group, or an alkylthio group having 1 to 6 carbon atoms. R₁ and R₂ may be the same or different from each other.

Specific examples of the linear alkyl group having 1 to 6 carbon atoms include a methyl group, an ethyl group, an n-propyl group, an n-butyl group, an n-pentyl group, and an n-hexyl group. The branched alkyl group having 1 to 6 carbon atoms is a branched alkyl group having 3 to 6 carbon atoms. Specific examples thereof include an isopropyl group, an isobutyl group, an s-butyl group, a t-butyl group, an isopentyl group, an s-pentyl group, a t-pentyl group, an isohexyl group, an s-hexyl group, and a t-hexyl group. Specific examples of the aryl group include a phenyl group, an o-tolyl group, an m-tolyl group, a p-tolyl group, a xylyl group, a trimethylphenyl group, a tetramethylphenyl group, a 1-naphthyl group, and a 2-naphthyl group. Specific examples of the aralkyl group include a benzyl group, a phenylethyl group, a phenylpropyl group, and a methylbenzyl group. Specific examples of the alkylthio group having 1 to 6 carbon atoms include a methylthio group, an ethylthio group, a propylthio group, an isopropylthio group, a butylthio group, an isobutylthio group, a sec-butylthio group, a tert-butylthio group, a pentylthio group, and a hexylthio group.

It is more preferable that R₁ and R₂, which are the same or different from each other, are a hydrogen atom, an amino group, or an alkylthio group having 1 to 6 carbon atoms.

R₅ and R₆ are a hydrogen atom or a group represented by -(CH₂)ₘ-Si(OR)₃₋ₚ(OH)ₚ (R is a methyl group or an ethyl group, m is an integer of 1 to 18, and p is an integer of 0 to 3). R₅ and R₆ may be the same or different from each other.

It is preferable that at least one of R₅ or R₆ is a group represented by -(CH₂)ₘ-Si(OR)₃₋ₚ(OH)ₚ.

In addition, in the group represented by -(CH₂)ₘ-Si(OR)₃₋ₚ(OH)ₚ, it is preferable that m is an integer of 2 to 12, it is more preferable that m is an integer of 3 to 10, and it is still more preferable that m is an integer of 3 to 8.

X represents a phenylene group or a group represented by -NH- or -(CH₂)ₙ- (n is an integer of 0 to 12).

X is preferably a group represented by -(CH₂)ₙ-. In addition, it is more preferable that n is an integer of 0 to 10, and it is still more preferable that n is an integer of 0 to 8, which is particularly preferably an integer of 0 to 6.

The compound represented by Chemical Formula (IV) (triazole compound (IV)) includes the triazole compound (I), a hydrolyzate of the triazole compound (I), and the triazole compound (II). In the triazole compound (IV), a compound in which p of the group represented by -(CH₂)ₘ-Si(OR)₃₋ₚ(OH)ₚ of R₅ and/or R₆ is an integer of 1 to 3 is a species that is generated by hydrolyzing the triazole compound (I) in which p is 0. Any of these is suitable as a component of the coupling agent. In addition, as a compound in which p is an integer of 1 to 3 in the triazole compound (IV), for example, a compound obtained by hydrolyzing the triazole compound (I) in which p is 0 and then removing a volatile component can be used.

In using the coupling agent according to the present invention, the same surface treatment method as in the case of the conventional coupling agent can be adopted.

Examples of the surface treatment method include (a) a method of subjecting a base material to a spray coating with a treatment liquid obtained by diluting an appropriate amount of a coupling agent with an organic solvent, (b) a method of subjecting a base material to a spray coating with a treatment liquid obtained by diluting the same coupling agent with water and an organic solvent, (c) a method of subjecting a base material to a spray coating with a treatment liquid obtained by diluting the same coupling agent with water, (d) a method of immersing a base material in a treatment liquid obtained by diluting the same coupling agent with an organic solvent, (e) a method of immersing a base material in a treatment liquid obtained by diluting the same coupling agent with water and an organic solvent, and (f) a method of immersing a base material in a treatment liquid obtained by diluting the same coupling agent with water.

Examples of the organic solvent include:
hydrocarbon-based solvents such as benzene, toluene, xylene, heptane, hexane, cyclohexane, and n-octane;
halogenated hydrocarbon-based solvents such as dichloromethane, dichloroethane, carbon tetrachloride, chloroform, chlorobenzene, dichlorobenzene, and trichlorobenzene;
ketone-based solvents such as acetone, methyl ethyl ketone, and methyl isobutyl ketone;
ether-based solvents such as diethyl ether, tetrahydrofuran, dioxane, ethylene glycol monomethyl ether (methyl cellosolve), ethylene glycol monoethyl ether (ethyl cellosolve), ethylene glycol monobutyl ether, and diethylene glycol monobutyl ether; and
alcohol-based solvents such as methanol, ethanol, 1-propanol, 2-propanol, n-butyl alcohol, 2-butyl alcohol, tert-butyl alcohol, ethylene glycol, diethylene glycol, and propylene glycol.

Examples of the base material that is used in the present invention include a base material which is, for example, granule-shaped, needle-shaped, fiber-shaped, woven fabric-shaped, plate-shaped, foilshaped, or amorphous, formed from a metal, an inorganic material, a resin material, or the like.

Examples of the metal include copper, aluminum, titanium, nickel, tin, iron, silver, gold, and an alloy of these metals, and a plate, a foil, a plating film, and the like, which consist of these metals, can be used as the base material.

Specific examples of the alloy are not particularly limited as long as the alloy as a copper alloy contains copper. Examples thereof include alloys which are, for example, Cu-Ag-based, Cu-Te-based, Cu-Mg-based, Cu-Sn-based, Cu-Si-based, Cu-Mn-based, Cu-Be-Co-based, Cu-Ti-based, Cu-Ni-Si-based, Cu-Zn-Ni-based, Cu-Cr-based, Cu-Zr-based, Cu-Fe-based, Cu-Al-based, Cu-Zn-based, and Cu-Co-based.

In addition, examples of the other alloys include aluminum alloys (Al-Si alloy), nickel alloys (Ni-Cr alloy), and iron alloys (Fe-Ni alloy, stainless steel, or steel).

Among these metals, copper and a copper alloy are preferable.

Examples of the inorganic material include silicon, ceramic, an inorganic material used as a filler, and glass.

Specific examples thereof include silicon compounds such as silicon, silicon carbide, silica, glass, diatomaceous earth, calcium silicate, talc, glass beads, celite activated clay, bentonite, aluminosilicate, and mica, oxides such as alumina, zinc oxide, iron oxide, magnesium oxide, tin oxide, and titanium oxide, hydroxides such as magnesium hydroxide, aluminum hydroxide, and basic magnesium carbonate, carbonates such as calcium carbonate, zinc carbonate, hydrotalcite, and magnesium carbonate, sulfates such as barium sulfate and gypsum, titanium salts such as barium titanate, nitrides such as aluminum nitride and silicon nitride, and carbon fibers.

Among these inorganic materials, silicon, ceramic (alumina, silicon carbide, aluminum nitride, silicon nitride, barium titanate, and the like), and glass are preferable.

Examples of the resin material include nylon, an acrylate resin, an epoxy resin, a polybenzoxazole resin, a silicone resin, a polyimide resin, a bismaleimide resin, a maleimide resin, a cyanate resin, a polyphenylene ether resin, a polyphenylene oxide resin, a polybutadiene resin, an olefin resin, a fluorine-containing resin, a polyetherimide resin, a polyether ether ketone resin, and a liquid crystal resin. These resins may be mixed or modified with each other, thereby being combined.

Among these resin materials, a polyphenylene ether resin, a polyphenylene oxide resin, a liquid crystal resin, an acrylate resin, an epoxy resin, an olefin resin, a polybenzoxazole resin, a silicone resin, and a polyimide resin are preferable.

In the case where a base material is subjected to a surface treatment with the treatment liquid containing the coupling agent according to the present invention, the lipophilicity of the surface of the base material is increased, which makes it possible to improve the affinity (adhesive properties and adhesiveness) for a resin or the like.

It is noted that in order to further exhibit the effect of this treatment, the base material that has been subjected to the surface treatment may be further subjected to a heat treatment.

### (Surface treatment liquid)

### <Surface treatment liquid containing triazole compound represented by Chemical Formula (IV)>

The surface treatment liquid according to the present invention contains a triazole compound represented by Chemical Formula (IV) (triazole compound (IV)). In the triazole compound (IV), a compound in which p of the group represented by -(CH₂)ₘ-Si(OR)₃₋ₚ(OH)ₚ of R₅ and/or R₆ is an integer of 1 to 3 is a species that is generated by hydrolyzing, in the surface treatment liquid, a compound in which p is 0. All of these are suitable as a component of the surface treatment liquid. In addition, in the triazole compound (IV), the compound in which p is an integer of 1 to 3 can be used, for example, by removing a volatile component from the surface treatment liquid containing the triazole compound (I) in which p is 0, thereby being extracted from the surface treatment liquid.

In implementing the present invention, it is preferable to use a compound in which p is 0, as a raw material when preparing the surface treatment liquid. In the case where p is 0, that is, in the case where at least one of R₅ or R₆ is a group represented by -(CH₂)ₘ-Si(OR)₃, the above-described compound can be the same compound as the triazole compound (I) described above, and the same applies to the preferred compound.

The surface treatment liquid according to the present invention is prepared by mixing the triazole compound (IV) and water.

As the water to be used for preparing the surface treatment liquid, pure water such as ion exchange water or distilled water is preferable.

In addition, in order to promote the dissolution (aqueous solution formation) of the triazole compound (IV), it is preferable to use a solubilizing agent in the surface treatment liquid according to the present invention. Examples of the solubilizing agent include an acid, an alkali, and an organic solvent. One kind of these solubilizing agents may be used alone, or two or more kinds thereof may be used in combination.

It is noted that regarding the preparation method for the surface treatment liquid in the case where a solubilizing agent and water are used, the solubilizing agent may be added after mixing the triazole compound with water, the triazole compound may be mixed with a mixed liquid of water and the solubilizing agent, or water may be added after mixing the triazole compound with the solubilizing agent.

Examples of the acid include inorganic acids such as hydrochloric acid, sulfuric acid, nitric acid, and phosphoric acid, and organic acids such as formic acid, acetic acid, propionic acid, butyric acid, 2-ethylbutyric acid, valeric acid, caproic acid, enanthic acid, caprylic acid, pelargonic acid, capric acid, lauric acid, myristic acid, palmitic acid, margaric acid, oleic acid, stearic acid, glycolic acid, lactic acid, gluconic acid, glyceric acid, malonic acid, succinic acid, levulinic acid, benzoic acid, oxalic acid, tartaric acid, malic acid, benzenesulfonic acid, tosylic acid, methanesulfonic acid, 5-sulfosalicylic acid, 4-hydroxybenzenesulfonic acid, 3-methyl-4-hydroxybenzenesulfonic acid, 4-aminobenzenesulfonic acid, camphorsulfonic acid, benzenedisulfonic acid, benzentrisulfonic acid, sulfamic acid, and amino acid. One kind of these acids may be used alone, or two or more kinds thereof may be used in combination.

Examples of the alkali include hydroxides of alkali metals such as sodium hydroxide and potassium hydroxide, and amines such as ammonia, methylamine, dimethylamine, trimethylamine, ethylamine, diethylamine, triethylamine, propylamine, isopropylamine, butylamine, pentylamine, hexylamine, heptylamine, octylamine, nonylamine, allylamine, ethylenediamine, diethylenetriamine, triethylenetetramine, monoethanolamine, diethanolamine, triethanolamine, monopropanolamine, dipropanolamine, tripropanolamine, monoisopropanolamine, diisopropanolamine, triisopropanolamine, 2-amino-1-propanol, N, N-dimethylethanolamine, cyclohexylamine, aniline, pyrrolidine, piperidine, piperazine, and pyridine. One kind of these alkalis may be used alone, or two or more kinds thereof may be used in combination.

Examples of the organic solvent include methanol, ethanol, 1-propanol, 2-propanol, butanol, tert-butyl alcohol, ethylene glycol, propylene glycol, 1,4-butanediol, glycerin, diethylene glycol, triethylene glycol, ethylene glycol monomethyl ether, ethylene glycol dimethyl ether, ethylene glycol monoethyl ether, ethylene glycol diethyl ether, ethylene glycol monobutyl ether, propylene glycol monomethyl ether, propylene glycol monoethyl ether, propylene glycol monobutyl ether, diethylene glycol monomethyl ether, diethylene glycol dimethyl ether, diethylene glycol monoethyl ether, diethylene glycol diethyl ether, diethylene glycol monobutyl ether, triethylene glycol dimethyl ether, triethylene glycol diethyl ether, tetrahydrofurfuryl alcohol, furfuryl alcohol, acetone, tetrahydrofuran, dioxane, acetonitrile, 2-pyrrolidone, formamide, dimethylformamide, dimethylacetamide, dimethyl sulfoxide, sulfolane, dimethyl carbonate, ethylene carbonate, N-methylpyrrolidone, γ-butyrolactone, and 1,3-dimethyl-2-imidazolidinone. One kind of these organic solvents may be used alone, or two or more kinds thereof may be used in combination.

In the surface treatment liquid, the content of the solubilizing agent is preferably 0.1% to 99% by weight, more preferably 0.5% to 99% by weight, and still more preferably 1% to 99% by weight.

### <Hydrolysis of triazole compound>

Since the triazole compound (IV) according to the present invention has a triazole ring in the molecule, the triazole ring interacts with the surface of the base material (metal, resin material, or inorganic material) to form a chemical bond, which makes it possible to improve the adhesiveness between different materials.

An aspect of the hydrolysis of the triazole compound (IV) is shown in a scheme (B). The scheme (B) exemplarily shows the case where at least one of R₅ or R₆ of the triazole compound (IV) is a group represented by -(CH₂)ₘ-Si(OR)₃₋ₚ(OH)ₚ.

In the scheme (B), an aspect in which the alkoxysilyl group contained in the triazole compound (IV) is hydrolyzed, that is, an aspect in which the trialkoxysilyl group is gradually changed to a dialkoxyhydroxysilyl group, a dihydroxyalkoxysilyl group, and a trihydroxysilyl group is shown.

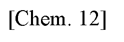

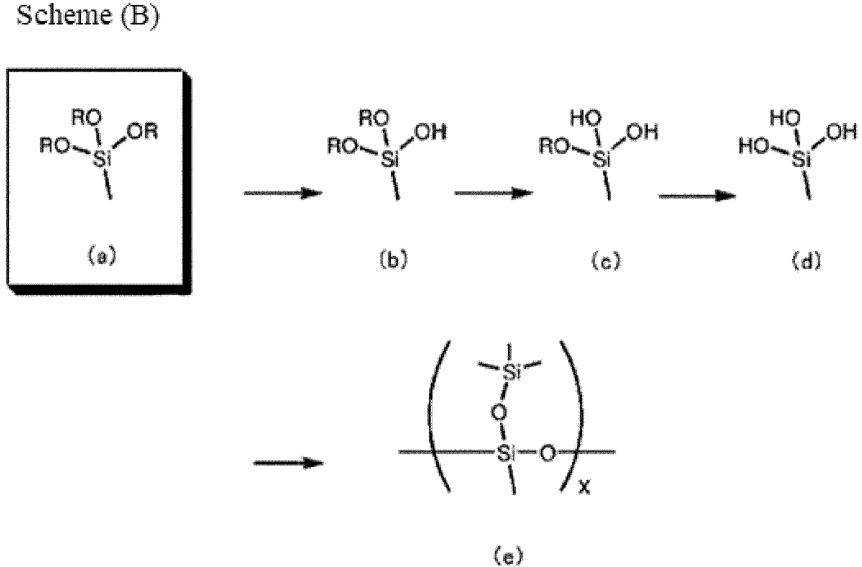

In general, a substance having an alkoxysilyl group in the molecule is known to act as a silane coupling agent.

For example, the adhesion between copper and a resin material as an example is as follows: in the case where the triazole compound that is used in the implementation of the present invention has an alkoxysilyl group in the molecule, the triazole ring chemically interacts with a resin and copper to form a chemical bond, the alkoxysilyl group (-Si-OR) is hydrolyzed to be converted into a hydroxysilyl group (-Si-OH), and this hydroxysilyl group is chemically bonded to the copper oxide that is scatteredly present on the surface of copper.

Therefore, in the case where, by bringing copper into contact with the surface treatment liquid, a chemical conversion coating derived from the triazole compound (IV) is formed on the surface of the copper due to the bonding to the triazole ring or the hydroxysilyl group, and when a resin layer consisting of a resin material is formed on the surface of the chemical conversion coating, the adhesiveness between the copper and the resin material can be enhanced as compared with a case where the resin layer is directly formed on the surface of the copper.

In implementing the present invention, the concentration of the triazole compound (IV) in the surface treatment liquid is preferably 0.0001 to 1.0000 mol/L, more preferably 0.0010 to 0.5000 mol/L, and still more preferably 0.0100 to 0.1000 mol/L, in terms of the concentration of the triazole compound as a trialkoxy form.

By the way, the triazole silane compound (IV) having a hydroxysilyl group, which is generated in the surface treatment liquid, gradually reacts with each other to undergo dehydration condensation, and the hydroxysilyl group forms a siloxane bond (Si-O-Si) (see the scheme (B)), whereby the triazole silane compound (IV) is converted into a silane oligomer which is difficult to dissolve in water (a triazole silane compound having a group represented by Chemical Formula (e) in the scheme (B)). It is noted that X in the group represented by Chemical Formula (e) is an integer that indicates the number of repeating units.

In the case where the amount of the silane oligomer generated in the surface treatment liquid becomes large, the undissolved fraction is precipitated (the treatment liquid is white turbid) and then adheres to a treatment tank, a pipe connected to the treatment tank, or sensors for detecting a temperature or a liquid surface of the treatment liquid, where the sensors are immersed in the treatment liquid, which may inhibit a smooth surface treatment.

In order to avoid this, in the preparation of the surface treatment liquid, it is preferable that the above-described organic solvent is contained in the surface treatment liquid as a solubilizing agent for the silane oligomer that is hardly soluble in water. In addition, in the preparation of the surface treatment liquid, it is preferable that the above-described solubilizing agent (acid, alkali, or organic solvent) is contained in order to promote the dissolution of the triazole compound. It is noted that since the organic solvent also has a function as a solubilizing agent for the silane oligomer, it is preferable that the surface treatment liquid according to the present invention contains, as the solubilizing agent, at least one selected from the group consisting of an acid, an alkali, and an organic solvent.

Similarly, in order to improve the stability of the surface treatment liquid and the uniformity of the chemical conversion coating, it is also possible to use a substance that generates halide ions such as a chloride ion, a bromide ion, and an iodide ion, or metal ions such as a copper ion, an iron ion, and a zinc ion.

The halide ion exhibits an effect of uniformly forming a flat part of the chemical conversion coating. Examples of the substance that generates a halide ion include lithium fluoride, sodium fluoride, potassium fluoride, magnesium fluoride, calcium fluoride, lithium chloride, sodium chloride, potassium chloride, magnesium chloride, calcium chloride, lithium bromide, sodium bromide, potassium bromide, magnesium bromide, calcium bromide, lithium iodide, sodium iodide, potassium iodide, magnesium iodide, calcium iodide, ammonium fluoride, ammonium chloride, ammonium bromide, ammonium iodide, cuprous chloride, cupric chloride, cuprous bromide, and cupric bromide. The halogen compound may be contained as an impurity of other components.

The content of the halide ion in the surface treatment liquid is not particularly limited. It is, for example, preferably 0.10 mol/L or less (particularly 0 to 0.10 mol/L), more preferably 0.050 mol/L or less (particularly 0 to 0.050 mol/L), still more preferably 0.020 mol/L or less (particularly 0 to 0.020 mol/L), and particularly preferably 0.010 mol/L or less (particularly 0 to 0.010 mol/L).

The copper ion can form a complex with a triazole compound, thereby increasing the strength of the chemical conversion coating or increasing the adhesive strength between the metal and the resin. The valence of the copper ion may be monovalent or divalent. Examples of the substance that generates a copper ion include metallic copper, copper sulfate (and a hydrate thereof (particularly, a pentahydrate)), copper formate (and a hydrate thereof (particularly, a tetrahydrate)), copper nitrate, cuprous chloride, cupric chloride, copper acetate (and a hydrate thereof (particularly, a monohydrate)), copper hydroxide, copper oxide, copper sulfide, copper carbonate, cuprous bromide, cupric bromide, copper phosphate, and copper benzoate.

In addition, the copper ion in the surface treatment liquid may include a copper ion eluted from metallic copper or copper oxide which is contained in a copper circuit, during the treatment of the copper circuit with the surface treatment liquid.

The content of the copper ion in the surface treatment liquid is not particularly limited. It is for example, preferably 1.00 mol/L or less (particularly 0 mol/L or more and 1.00 mol/L or less), more preferably 0.50 mol/L or less (particularly 0 mol/L or more and 0.50 mol/L or less), still more preferably 0.10 mol/L or less (particularly 0 mol/L or more and 0.10 mol/L or less), and particularly preferably 0.010 mol/L or less (particularly 0 mol/L or more and 0.010 mol/L or less).

In addition, a publicly known coupling agent may be used in combination within a range in which the effect of the present invention is not impaired. Examples of the publicly known coupling agent include silane-based coupling agents (silane coupling agents) having a thiol group (mercapto group), a vinyl group, an epoxy group, a (meth)acryl group, an amino group, a chloropropyl group, or the like.

Examples of such a silane-based coupling agent include the following compounds:
mercaptosilane compounds such as:
   3-mercaptopropyltrimethoxysilane, and
   3-mercaptopropylmethyldimethoxysilane,
vinyl silane compounds such as:
   vinyl trichlorosilane,
   vinyl trimethoxysilane, and
   vinyl triethoxysilane,
styrylsilane compounds such as p-styryltrimethoxysilane,
epoxy silane compounds such as:
   2-(3,4-epoxycyclohexyl)ethyltrimethoxysilane,
   3-glycidoxypropyltrimethoxysilane,
   3-glycidoxypropylmethyldiethoxysilane, and
   3-glycidoxypropyltriethoxysilane,
acryloxysilane compounds such as 3-acryloxypropyltrimethoxysilane,
methacryloxysilane compounds such as:
   3-methacryloxypropylmethyldimethoxysilane,
   3-methacryloxypropyltrimethoxysilane,
   3-methacryloxypropylmethyldiethoxysilane, and
   3-methacryloxypropyltriethoxysilane,
amino silane compounds such as:
   N-2-(aminoethyl)-3-aminopropylmethyldimethoxysilane,
   N-2-(aminoethyl)-3-aminopropyltrimethoxysilane,
   N-2-(aminoethyl)-3-aminopropyltriethoxysilane,
   3-aminopropyltrimethoxysilane,
   3 -aminopropyltriethoxysilane,
   3-triethoxysilyl-N-(1,3-dimethyl-butylldene)propylamine,
   N-phenyl-3-aminopropyltrimethoxysilane,
   N-(vinylbenzyl)-2-aminoethyl-3-aminopropyltrimethoxysilane,
ureidosilane compounds such as 3-ureidopropyltriethoxysilane,
chloropropyl silane compounds such as 3-chloropropyltrimethoxysilane,
sulfide silane compounds such as bis(triethoxysilylpropyl) tetrasulfide, and
isocyanatosilane compounds such as 3-isocyanatopropyltriethoxysilane.

In addition, examples thereof also include aluminum-based coupling agents, titanium-based coupling agents, and zirconium-based coupling agents.

### (Treatment method)

A method of bringing the surface treatment liquid according to the present invention into contact with the surface of the base material is not particularly limited, and a means such as spraying, immersion, or coating can be adopted as in the case of the above-described coupling agent.

The time (treatment time) during which the surface treatment liquid is in contact with the base material is preferably set to 1 second to 10 minutes, and more preferably set to 5 seconds to 3 minutes. In the case where the treatment time is less than 1 second, the film thickness of the chemical conversion coating formed on the surface of the base material is reduced, and thus it is hard to sufficiently obtain the adhesive force between materials having qualities different from each other. On the other hand, in the case where it is longer than 10 minutes, there is no significant difference in the film thickness of the chemical conversion coating. Therefore, it is preferable that the treatment time is within 10 minutes from the viewpoint of productivity.

In addition, the temperature of the treatment liquid when bringing the surface treatment liquid into contact with the surface of the base material is preferably set to 5°C to 50°C; however, it may be appropriately set in terms of the relationship to the treatment time.

After the surface treatment liquid according to the present invention is brought into contact with the base material, the base material may be washed with water and then dried, or may be dried without being washed with water.

For the drying, the temperature is preferably set to in a range of room temperature to 150°C.

It is noted that although the water to be used for the washing with water is preferably pure water such as ion exchange water or distilled water, a method and a time for the washing with water are not particularly limited, and thus an appropriate time with a means such as spraying or immersion may be adopted.

It is noted that the film thickness of the chemical conversion coating is preferably 0.5 to 1,000 nm, more preferably 1 to 200 nm, and still more preferably 1 to 100 nm. In the case where the film thickness is 0.5 nm or more, the adhesiveness between materials is sufficiently increased, and in the case where the film thickness is 1,000 nm or less, the chemical resistance of the chemical conversion coating can be maintained.

In the present invention, the chemical conversion coating after drying may be subjected to a treatment by plasma, a laser, an ion beam, ozone, heating, humidification, or the like to modify the surface of the chemical conversion coating. Alternatively, the washing of the metal surface may be carried out for the purpose of removing the resin and ion residues on the metal surface by using plasma, a laser, an ion beam, a mechanical polishing with pumice brushing, a processing method with a drill, or the like.

Before bringing the surface treatment liquid according to the present invention into contact with the surface of copper or a copper alloy (hereinafter, both may be simply referred to as copper), the surface of the copper may be subjected to at least one pretreatment selected from an acid washing treatment, an alkali treatment, a roughening treatment, a treatment for heat resistance, a rust prevention treatment, or a chemical conversion treatment.

The acid washing treatment is a treatment that is carried out in order to remove an oil and fat component that has adhered to the surface of copper and in order to remove an oxide coating film on the surface of copper. For the acid washing treatment, a solution such as a hydrochloric acid-based solution, a sulfuric acid-based solution, a nitric acid-based solution, a sulfuric acid-hydrogen peroxide-based solution, an organic acid-based solution, an inorganic acid-organic solvent-based solution, or an organic acid-organic solvent-based solution can be used.

The alkali treatment is a treatment that is carried out in order to remove an oil and fat component that has adhered to the surface of copper or in order to remove a residue (for example, a dry film resist for forming a copper circuit) of the previous step. For the alkali treatment, it is possible to use a solution such as an aqueous solution or organic solvent-based solution containing an alkali metal hydroxide such as sodium hydroxide or potassium hydroxide, an amine such as ammonia, ethanolamine, monopropanolamine, or tetramethylammonium hydroxide, sodium carbonate, sodium hydrogen carbonate, potassium carbonate, potassium hydrogen carbonate, ammonium carbonate, ammonium hydrogen carbonate, sodium acetate, potassium acetate, sodium phosphate, disodium hydrogen phosphate, potassium phosphate, dipotassium hydrogen phosphate, or the like.

The roughening treatment is a treatment that is carried out in order to enhance the adhesiveness between the copper and the resin due to the anchor effect, and an uneven shape is provided on the surface of the copper, which makes it possible to enhance the adhesiveness between the copper and the resin material. In this roughening treatment, a method such as a micro-etching method, an electroplating method, an electroless plating method, an oxidation method (black oxide or brown oxide), an oxidation/reduction method, a brush polishing method, or a jet scrubbing method can be adopted.

In the micro-etching method, for example, any of organic acid-cupric ion-based etchants, sulfuric acid-hydrogen peroxide-based etchants, persulfate-based etchants, copper chloride-based etchants, and iron chloride-based etchants can be used. In the electroplating method, unevenness is provided on the surface of copper by precipitating fine copper particles on the surface of copper.

In the treatment for heat resistance, a coating formed from at least one selected from nickel, nickel-phosphorus, zinc, zinc-nickel, copper-zinc, copper-nickel, copper-nickel-cobalt, or nickel-cobalt is formed on the surface of the copper. The formation of the coating can be carried out by adopting a publicly known method using electroplating; however, the method is not limited to electroplating, and there is no problem even in the case where vapor deposition or other methods are used.

The rust prevention treatment is carried out in order to prevent the surface of copper from oxidizing and corroding, and a method of forming a plating film of zinc or a zinc alloy composition, or a plating film of electrolytic chromate on the surface of copper can be adopted. In addition, a treatment liquid containing an organic compound-based anticorrosion agent such as a benzotriazole-based anticorrosion agent may be brought into contact with the surface of copper.

In the above-described chemical conversion treatment, a method of forming a passive state film of tin or a method of forming a passive state film of copper oxide can be adopted.

Before and/or after the surface treatment liquid according to the present invention is brought into contact with the surface of copper, an aqueous solution containing copper ion may be brought into contact with the surface of copper. This aqueous solution containing the copper ion has a function of enhancing the film formability of the chemical conversion coating that is formed on the surface of copper, or a function of making uniform the thickness of the chemical conversion coating that is formed on the surface of copper. The valence of the copper ion is not particularly limited, and the copper ion is a monovalent or divalent copper ion.

A copper ion source for the aqueous solution containing copper ion is not particularly limited as long as it is a copper salt that is soluble in water, and examples thereof include copper salts such as copper sulfate, copper nitrate, copper chloride, copper formate, and copper acetate. In order to dissolve a copper salt in water, ammonia, hydrochloric acid, or the like may be added.

Before and/or after the surface treatment liquid according to the present invention is brought into contact with the surface of copper, an acidic aqueous solution or an alkaline aqueous solution may be brought into contact with the surface of the copper. This acidic aqueous solution or alkaline aqueous solution also has a function of making uniform the thickness of the chemical conversion coating that is formed on the surface of copper as in the case of the aqueous solution containing copper ions.

The acidic aqueous solution and the alkaline aqueous solution are not particularly limited; however, examples of the acidic aqueous solution include an aqueous solution containing a mineral acid such as sulfuric acid, nitric acid, or hydrochloric acid, and an aqueous solution containing an organic acid such as formic acid, acetic acid, lactic acid, glycolic acid, or an amino acid. Examples of the alkaline aqueous solution include an aqueous solution containing an alkali metal hydroxide such as sodium hydroxide or potassium hydroxide, an amine such as ammonia, ethanolamine, monopropanolamine, or tetramethylammonium hydroxide, sodium carbonate, sodium hydrogen carbonate, potassium carbonate, potassium hydrogen carbonate, ammonium carbonate, ammonium hydrogen carbonate, sodium acetate, potassium acetate, sodium phosphate, disodium hydrogen phosphate, potassium phosphate, dipotassium hydrogen phosphate, or the like.

Before the surface treatment liquid according to the present invention is brought into contact with the surface of copper, an aqueous solution containing a publicly known coupling agent may be brought into contact with the surface of the copper.

After the surface treatment liquid according to the present invention is brought into contact with the surface of copper, an aqueous solution containing a publicly known coupling agent may be brought into contact with the surface of the copper.

After the surface treatment liquid according to the present invention is brought into contact with the surface of copper, a treatment liquid containing a publicly known organic compound-based anticorrosion agent, for example, a benzotriazole-based anticorrosion agent, may be brought into contact with the surface of the copper.

The surface treatment liquid according to the present invention can be used for treating a surface of at least one base material selected from the group consisting of the metal, the inorganic material, and the resin material. In the case of treating the surface of the base material with the surface treatment liquid according to the present invention, it is possible to form a chemical conversion coating on the surface of the base material and to enhance the adhesiveness with other materials.

In the present invention, two materials selected from the group consisting of the metal, the inorganic material, and the resin material can be adhered to each other by using the surface treatment liquid according to the present invention. The mutual affinity of two materials can be improved in the case where the two materials are adhered through a layer of the chemical conversion coating formed from the surface treatment liquid according to the present invention. As a result, it is possible to more firmly adhere even such materials having qualities different from each other.

### (Adhesion method)

An adhesion method for two materials can be carried out according to a publicly known method. Examples thereof include a method of bringing the surface treatment liquid according to the present invention into contact with a surface of a base material composed of a metal, an inorganic material, or a resin material, thereby forming a chemical conversion coating on the surface thereof, and adhering another base material to a part or the whole of the formed chemical conversion coating by using a means such as coating, pressure bonding, or mixing, or using an adhesive or an adhesive sheet (film), or by combining these means.

In addition, examples thereof include a method of bringing the surface treatment liquid according to the present invention into contact with a surface of each of two base materials selected from a metal, an inorganic material, and a resin material, thereby forming a chemical conversion coating on the surface of each of the two base materials, and adhering the two base materials to each other by using a means such as coating, pressure bonding, or mixing, or using an adhesive or an adhesive sheet (film), or by combining these means.

By using the surface treatment liquid according to the present invention, it is possible to mutually adhere such two materials as described above, particularly, two materials having qualities different from each other. Therefore, the surface treatment liquid according to the present invention can be suitably used for various electronic devices such as various electrical and electronic components, semiconductor wafers, and printed wiring boards.

It is noted that in the present invention, the surface treatment liquid according to the present invention can be suitably used for a base material formed from a metal, particularly copper or a copper alloy. For example, it is suitable for a surface treatment of copper or a copper alloy, which aims to improve adhesive properties (adhesiveness) between a copper circuit (copper wiring layer) and a prepreg or a solder resist, which is semi-cured or cured, or a dry film resist (insulating resin layer) which is semi-cured or cured. Also, in a printed wiring board that has an insulating resin layer in contact with a copper wiring layer, the adhesive properties between the copper wiring layer and the insulating resin layer can be improved.

In a use example in a semiconductor wafer, it is suitable for a surface treatment of a semiconductor circuit, which aims to enhance adhesive properties (adhesiveness) between a semiconductor circuit formed on a semiconductor wafer and a protective film (for example, an insulating protective film such as a photosensitive positive-tone buffer coat, a photosensitive negative-tone buffer coat, or a nonphotosensitive buffer coat, or a bump protective film).

In addition, in a package substrate (WL-CSP, FO-WLP, PLP) in which a rewiring layer is formed on a semiconductor wafer, or a two-and-a-half dimensional (2.5D) or three-dimensional (3D) interposer substrate, it is suitable for a surface treatment of a copper circuit rewiring layer, which aims to enhance adhesive properties (adhesiveness) between a copper circuit rewiring layer and an insulating material.

Examples of the protective film or the insulating material include a polyimide resin, a polybenzoxazole resin, and a silicone resin.

The printed wiring board can be produced by bringing the surface treatment liquid according to the present invention into contact with the surface of the copper wiring line and then carrying out washing with water and drying to form an insulating resin layer on the surface of the copper wiring line. This method of contact is as described above, and immersion of the copper wiring line in the surface treatment liquid, spraying of the treatment liquid onto the copper wiring line, or the like is simple and reliable, which is preferable.

In addition, the method for washing with water described above is not particularly limited; however, immersion of the copper wiring line in the washing water or spraying of the washing water onto the surface of the copper wiring line is simple and reliable, which is preferable.

For the formation of the insulating resin layer, a publicly known method, for example, a method of attaching a semi-cured resin material, a means for applying a liquid resin material containing a solvent, or the like can be adopted. Next, a via hole is formed in order to allow the upper and lower wiring lines to be conductive. By repeating this process, a multilayer printed wiring board can be produced.

In the method for forming a circuit of a printed wiring board, an example of a semi-additive method using the surface treatment liquid according to the present invention is shown below.

In a manufacturing method for a circuit board including at least one or more of the following steps of:
(a) a step of preparing an insulating substrate that has a first conductive layer on a first surface of an insulating substrate or an insulating substrate having a penetrating hole as a through-hole and a via hole, on a second surface opposite the first surface as well as on an inner wall of the penetrating hole as a through-hole and on an inner wall of the via hole,
(b) a step of forming a photo-crosslinkable resin layer and a mask layer on the first surface and on the second surface to cover the first conductive layer on the first surface, on the second surface as well as on the inner wall of the penetrating hole as a through-hole and on the inner wall of the via hole, with the photo-crosslinkable resin layer and the mask layer,
(c) a step of patternwise exposing the photo-crosslinkable resin layer on the first surface and the second surface as well as in the periphery of the penetrating hole as a through-hole and the via hole,
(d) a step of removing the mask layer on the first surface and the second surface as well as in the periphery of the penetrating hole as a through-hole and the via hole,
(e) a step of developing and removing, by using a photo-crosslinkable resin layer removal liquid, an uncured photo-crosslinkable resin layer on the first surface and the second surface as well as in the periphery of the penetrating hole as a through-hole and the via hole, and exposing the first conductive layer on the first surface and the first conductive layer on the second surface, as well as the first conductive layer in the periphery of the penetrating hole as a through-hole and the via hole,
(f) a step of forming a second conductive layer, by an electrolytic plating treatment, on the first conductive layer exposed on the first surface and the second surface as well as on the inner wall of the penetrating hole as a through-hole and the via hole,
(g) a step of removing a cured photo-crosslinkable resin layer on the first surface and on the second surface as well as in the periphery of the penetrating hole as a through-hole and the via hole, thereby exposing the first and second conductive layers on the first surface and the second surface as well as on the inner wall of the penetrating hole as a through-hole and the via hole,
(h) a step of removing the exposed first conductive layer by flash etching,
(i) a step of forming a third conductive layer, by electroless plating and electrolytic plating treatments, on the first and second conductive layers on the first surface and the second surface as well as on the inner wall of the penetrating hole as a through-hole and the via hole, and
(j) a step of laminating an insulating resin layer on the first, second, and third conductive layers on the first surface and the second surface as well as on the inner wall of the penetrating hole as a through-hole and the via hole,
the surface treatment liquid according to the present invention is brought into contact with at least one or more of the metal layers or resist layers in the first, second, and third conductive layers on the first surface and the second surface as well as on the inner wall of the penetrating hole as a through-hole and the via hole, in the insulating resin substrate, in the photo-crosslinkable resin layer used for the etching resist layer or the plating resist layer, and in the insulating resin layer, whereby a printed wiring board is manufactured.

Further, in the method for forming a circuit of a printed wiring board, an example of a subtractive method using the surface treatment liquid according to the present invention is shown below.

In a manufacturing method for a circuit board including at least one or more of the following steps of:
(a) a step of preparing an insulating substrate that has a first conductive layer on a first surface of an insulating substrate or an insulating substrate having a penetrating hole as a through-hole and a via hole, on a second surface opposite the first surface as well as on an inner wall of the penetrating hole as a through-hole and on an inner wall of the via hole,
(b) a step of forming a photo-crosslinkable resin layer and a mask layer on the first surface and on the second surface to cover the first conductive layer on the first surface, on the second surface as well as on the inner wall of the penetrating hole as a through-hole and on the inner wall of the via hole, with the photo-crosslinkable resin layer and the mask layer,
(c) a step of patternwise exposing the photo-crosslinkable resin layer on the first surface and the second surface as well as in the periphery of the penetrating hole as a through-hole and the via hole,
(d) a step of removing the mask layer on the first surface and the second surface as well as in the periphery of the penetrating hole as a through-hole and the via hole,
(e) a step of developing and removing, by using a photo-crosslinkable resin layer removal liquid, an uncured photo-crosslinkable resin layer on the first surface and the second surface as well as in the periphery of the penetrating hole as a through-hole and the via hole, and exposing the first conductive layer on the first surface and the first conductive layer on the second surface, as well as the first conductive layer in the periphery of the penetrating hole as a through-hole and the via hole,
(f) a step of etching the first conductive layer exposed on the first surface and the second surface as well as on the inner wall of the penetrating hole as a through-hole and the via hole, thereby removing the exposed first conductive layer,
(g) a step of removing a cured photo-crosslinkable resin layer on the first surface and on the second surface as well as in the periphery of the penetrating hole as a through-hole and the via hole, thereby exposing the first and second conductive layers on the first surface and the second surface as well as on the inner wall of the penetrating hole as a through-hole and the via hole,
(h) a step of forming a third conductive layer, by electroless plating and electrolytic plating treatments, on the first and second conductive layers on the first surface and the second surface as well as on the inner wall of the penetrating hole as a through-hole and the via hole, and
(i) a step of laminating an insulating resin layer on the first, second, and third conductive layers on the first surface and the second surface as well as on the inner wall of the penetrating hole as a through-hole and the via hole,
the surface treatment liquid according to the present invention is brought into contact with at least one or more of the metal layers or resist layers in the first, second, and third conductive layers on the first surface and the second surface as well as on the inner wall of the penetrating hole as a through-hole and the via hole, in the insulating resin substrate, in the photo-crosslinkable resin layer used for the etching resist layer or the plating resist layer, and the insulating resin layer, whereby a printed wiring board is manufactured.

The copper wiring line or the conductive layer may be such as one that is produced by any method such as an electroless plating method, an electrolytic plating method, a vapor deposition method, a sputtering method, or a damascene method, and it may be such as one that includes an inner via hole, a through-hole, a connection terminal, or the like.

In addition, the "copper" according to the present invention is such as one that is used in use applications and forms such as a foil (an electrolytic copper foil, a rolled copper foil, a resin-attached copper foil, a carrier-attached copper foil, an electroless copper foil, a sputtered copper foil, or a thin copper foil) which is used for an electronic device such as a printed wiring board or a lead frame, a decoration, a building material, or the like, a plating film (an electroless copper plating film or an electrolytic copper plating film), a thin film formed by a vapor deposition method, a sputtering method, a damascene method, or the like, a particle, a needle, a fiber, a wire, a rod, a tubes, and a plate. It is noted that in the case of a copper wiring line in recent years through which a high-frequency electrical signal flows, the surface of the copper is preferably a smooth surface having an average roughness of 0.1 µm or less. As a pretreatment, the surface of copper may be subjected to plating with nickel, zinc, chromium, tin, or the like.

In addition, for example, in a use example in a lead frame in the case of being subjected to wire bonding mounting, the surface treatment liquid according to the present invention is suitable for a surface treatment of a lead frame, which aims to enhance adhesive properties (adhesiveness) to a sealing resin or an adhesive when mounting a semiconductor chip, on a metal surface in a lead frame production step, a metal surface of a frame after mounting a semiconductor chip (before and after a die bonding and pre-baking step), a metal surface of a frame after being subjected to wire bonding mounting, or a metal surface of a frame in steps until resin sealing is carried out (before and after a resin molding and baking step).

In addition, for example, in a use example in a lead frame in the case of being subjected to flipchip mounting, the surface treatment liquid according to the present invention is suitable for a surface treatment of a lead frame, which aims to enhance adhesive properties (adhesiveness) to a sealing resin or an adhesive when mounting a semiconductor chip, on a metal surface in a lead frame production step, a metal surface of a lead frame after temporary placement of a bonding material (a material for solder, Au plating, Sn plating, or the like), a metal surface of a frame after mounting a semiconductor chip (before and after a position alignment and chip mounting/baking step), a metal surface of a lead frame after main curing (before and after a step with reflow heating, thermocompression bonding, ultrasonic waves, plasma, or the like), or a metal surface of a lead frame in steps until resin sealing is carried out.

In addition, for example, in a use example in a fine wiring board in which an integration technology for disposing semiconductor chips close to each other is enhanced, the surface treatment liquid according to the present invention is also suitable for a surface treatment of a copper circuit wiring layer, which aims to enhance adhesive properties (adhesiveness) between a copper circuit wiring layer and an insulating material, in a 2.1-dimensional (2.1D) organic substrate or glass substrate, a componentembedded substrate (EPS substrate) in which a semiconductor is embedded in a substrate, or a coreless substrate.

In addition, for example, the surface treatment liquid for metal, according to the present invention is suitable for a surface treatment of a copper circuit wiring layer, which aims to enhance adhesive properties (adhesiveness) between a copper circuit wiring layer and an insulating material, in the case of subjecting the patterned wiring line-embedded upper and lower layers to laser via processing and carrying out via-filling plating, or in the case of using a circuit-embedded substrate (ETS substrate) in which a copper pillar formed by plating is used for conduction between upper and lower layers and MIS that uses a mold resin is used for the insulating layer.

In addition, the carrier-attached copper foil that is treated with the surface treatment liquid according to the present invention is an extremely thin electrolytic copper foil that is used for printed wiring board, which includes a step of forming a circuit by any of a semi-additive method, a subtractive method, a partly additive method, and a modified semi-additive method. The carrier-attached copper foil includes a copper foil carrier, a peeling layer laminated on the copper foil carrier, and an extremely thin copper layer laminated on the peeling layer. The copper surface may be subjected to at least one pretreatment selected from the group consisting of an acid washing treatment, a roughening treatment, a treatment for heat resistance, a rust prevention treatment, and a chemical conversion treatment.

### (Insulating composition)

The coupling agent according to the present invention can be contained in a resin material or an inorganic material to obtain an insulating composition.

In addition, an insulating composition can be also obtained by dissolving the triazole compound (IV) in an organic solvent or the like and mixing the resultant solution with a resin material or an inorganic material.

The content of the triazole compound (IV) in the insulating composition is preferably 0.001% to 10% by weight and more preferably 0.01% to 5% by weight. In the case where the content of the triazole compound (IV) is 0.001% by weight or more in the insulating composition, the effect of improving the adhesive properties is sufficiently obtained. In the case where the concentration is more than 10% by weight, the effect of improving the adhesive properties almost reaches a plateau, and thus the content of the triazole compound (IV) is preferably 10% by weight or less from an economical viewpoint.

The insulating composition can be produced by a publicly known method. For example, an insulating composition can be produced by dissolving the triazole compound (IV) in an organic solvent and mixing the resultant solution with a solid or liquid resin material. In addition, the triazole compound (IV) may be directly added to and mixed with a liquid resin material to produce an insulating composition.

Since the insulating composition according to the present invention provides an insulating material having high adhesive strength, the insulating composition according to the present invention can be suitably used for various electrical and electronic components and electronic devices such as a printed wiring board.

By the way, JP2009-19266A discloses an invention relating to a forming method of a silane coupling agent film, the method including a step of applying a liquid containing a silane coupling agent onto a metal surface, a step of drying the metal surface to which the liquid has been applied, at a temperature of 25°C to 150°C and within 5 minutes, and a step of washing the dried metal surface with water.

In addition, it is said that a metal layer having adhesive properties such as tin may be formed in advance on the metal surface by an immersion plating liquid as a surface treatment.

The surface treatment liquid according to the present invention can be used as the above-described liquid containing a silane coupling agent. It is noted that the contents described in this patent publication are incorporated in the present specification by reference.

### EXAMPLES

Hereinafter, the present invention will be specifically described in reference to Examples (synthesis test and evaluation test); however, the present invention is not limited thereto.

It is noted that OEt in the following chemical formulae represents an ethoxy group.

The triazole compounds and the halogenated alkyl silane compound, which were used as raw materials in the synthesis test, are as follows.

### [Triazole compound]

· Triazole compound (3,3'-bis(5-amino-1,2,4-triazole)) represented by Formula (11): synthesized in accordance with the method described in "Chem. Eur. J.", Vol, 18, p. 16742 (2012).
· Triazole compound (3,3'-methylene bis(5-amino-1,2,4-triazole)) represented by Formula (12): synthesized in accordance with the method described in "Russ. J. Applied. Chem.", Vol. 82, p. 276 (2009).
· Triazole compound (3,3'-tetramethylene bis(5-amino-1,2,4-triazole)) represented by Formula (13): synthesized in accordance with the method described in the specification of United States Patent No. 2744116.
· Triazole compound (3,3'-bis(5-methyl-1,2,4-triazole)) represented by Formula (14): synthesized in accordance with the method described in "Indian J. Chem.", Vol. 44B, p. 568 (2005).

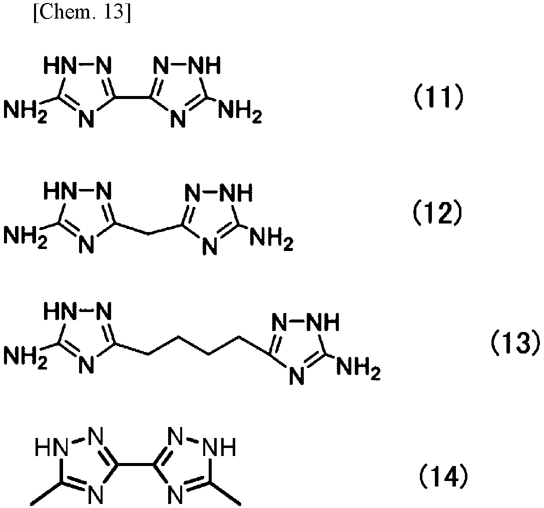

### [Halogenated alkyl silane compound]

· 3-chloropropyltriethoxysilane: manufactured by Tokyo Chemical Industry Co., Ltd.

The triazole compounds used in Comparative Example 1 (evaluation test) are as follows.

### [Triazole compound]

· Triazole compound (3-amino-5-methyl-1,2,4-triazole) represented by Formula (21): synthesized in accordance with the method described in the specification of United States Patent No. 4734413.
· Triazole compound (3-amino-5-methyl-1-[3-(triethoxysilyl)propyl]-1,2,4-triazole) represented by Formula (22): synthesized in accordance with the method described in International Publication No. WO2018/186476A.

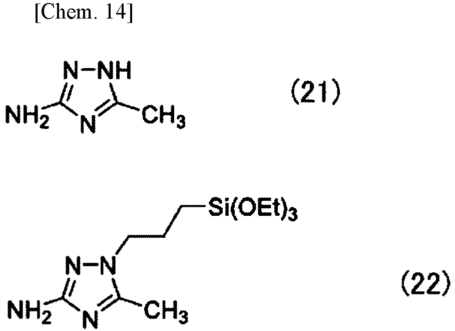

### <Synthesis of triazole compound (I)>

Triazole compounds of Examples 1 to 5 were synthesized.

### [Example 1]

### <Synthesis of mixture of 1-[3-(triethoxysilyl)propyl]-3,3'-bis(5-amino-1,2,4-triazole) and 2-[3-(triethoxysilyl)propyl]-3,3'-bis(5-amino-1,2,4-triazole)>

A compound represented by Formula (1) (mixture of 1-[3-(triethoxysilyl)propyl]-3,3'-bis(5-amino-1,2,4-triazole) and 2-[3-(triethoxysilyl)propyl]-3,3'-bis(5-amino-1,2,4-triazole)) was synthesized.

A suspension consisting of 7.80 g (46.9 mmol) of 3,3'-bis(5-amino-1,2,4-triazole) and 200 mL of N,N-dimethylacetamide was heated to 80°C, 16.20 g (47.6 mmol) of a 20% sodium ethoxide ethanol solution was added thereto, and the temperature was raised to 100°C, followed by stirring for 1 hour. Next, the temperature was set to 70°C, and then 11.40 g (47.3 mmol) of 3-chloropropyltriethoxysilane was added thereto, followed by stirring for 1 hour. Subsequently, the temperature was raised to 100°C, followed by stirring for 16.5 hours. After cooling the reaction solution to 60°C, insoluble matters were filtered off, and the filtrate was distilled off under reduced pressure to obtain 15.81 g of a brown candy-like substance (46.9 mmol, yield: 91.0%).

The ¹H-NMR spectral data of the obtained brown candy-like substance were as follows.

¹H-NMR (DMSO-d₆) δ: 0.55 (m, 2H), 1.14 (m, 9H), 1.75 (m, 2H), 3.34 (m, 2H), 3.73 (m, 6H), 5.23 (s, 2H), 6.41 (s, 2H).

From this ¹H-NMR spectral data, the obtained brown candy-like substance was identified as a triazole compound represented by Chemical Formula (1).

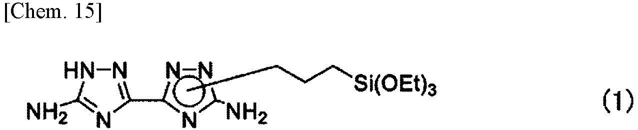

### [Example 2]

### <Synthesis of mixture of 1-[3-(triethoxysilyl)propyl]-3,3'-methylene bis(5-amino-1,2,4-triazole) and 2-[3-(triethoxysilyl)propyl]-3,3'-methylene bis(5-amino-1,2,4-triazole)>

A compound represented by Formula (2) (mixture of 1-[3-(triethoxysilyl)propyl]-3,3'-methylene bis(5-amino-1,2,4-triazole) and 2-[3-(triethoxysilyl)propyl]-3,3'-methylene bis(5-amino-1,2,4-triazole)) was synthesized.

A suspension consisting of 6.08 g (33.8 mol) of 3,3'-methylene bis(5-amino-1,2,4-triazole) and 150 mL of N,N-dimethylacetamide was heated to 80°C, 11.72 g (34.4 mol) of a 20% sodium ethoxide ethanol solution was added thereto, followed by stirring for 2 hours. Then, 8.30 g (34.5 mol) of 3-chloropropyltriethoxysilane was added thereto, followed by stirring for 30 minutes and then stirring at 100°C for 20 hours. After cooling the reaction solution to 70°C, insoluble matters were filtered off, and the filtrate was distilled off under reduced pressure to obtain 10.50 g of a light brown solid (27.3 mol, yield: 80.8%).

The ¹H-NMR spectral data of the obtained light brown solid were as follows.

¹H-NMR (DMSO-d₆) δ: 0.57 (m, 2H), 1.15 (m, 9H), 1.53 (m, 2H), 2.94 (s, 2H), 3.45 (m, 2H), 3.73 (m, 6H), 5.04 (s, 2H), 5.98 (s, 2H).

From this ¹H-NMR spectral data, the obtained light brown solid was identified as a triazole compound represented by Chemical Formula (2).

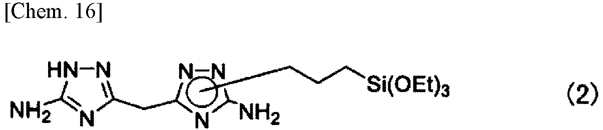

### [Example 3]

### <Synthesis of mixture of 1-[3-(triethoxysilyl)propyl]-3,3'-tetramethylene bis(5-amino-1,2,4-triazole) and 2-[3-(triethoxysilyl)propyl]-3,3'-tetramethylene bis(5-amino-1,2,4-triazole)>

A compound represented by Formula (3) (mixture of 1-[3-(triethoxysilyl)propyl]-3,3'-tetramethylene bis(5-amino-1,2,4-triazole) and 2-[3-(triethoxysilyl)propyl]-3,3'-tetramethylene bis(5-amino-1,2,4-triazole)) was synthesized.

A suspension consisting of 8.20 g (36.9 mol) of 3,3'-tetramethylene bis(5-amino-1,2,4-triazole) and 200 mL of N,N-dimethylacetamide was heated to 80°C, 12.70 g (37.3 mol) of a 20% sodium ethoxide ethanol solution was added thereto, and the temperature was raised to 100°C, followed by stirring for 1 hour. Next, the temperature was set to 70°C, and then 9.04 g (37.5 mol) of 3-chloropropyltriethoxysilane was added thereto, followed by stirring for 1 hour. Subsequently, the temperature was raised to 100°C, followed by stirring for 16 hours. After cooling the reaction solution to 60°C, insoluble matters were filtered off, and the filtrate was distilled off under reduced pressure to obtain 13.8 g of a light brown candy-like substance (32.3 mol, yield: 87.7%).

The ¹H-NMR spectral data of the obtained light brown candy-like substance were as follows.

¹H-NMR (DMSO-d₆) δ: 0.50 (m, 2H), 1.13 (t, 9H), 1.61 (m, 4H), 2.09 (m, 2H), 2.42 (m, 4H), 3.34 (m, 2H), 3.73 (m, 6H), 5.06 (s, 2H), 5.96 (s, 2H).

From this ¹H-NMR spectral data, the obtained light brown candy-like substance was identified as a triazole compound represented by Chemical Formula (3).

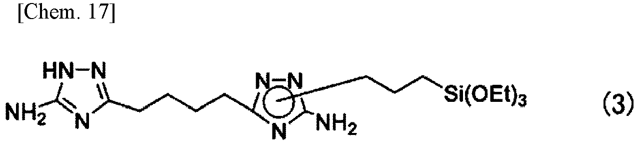

### [Example 4]

### <Synthesis of mixture of 3,3'-bis{1-[3-(triethoxysilyl)propyl]-5-amino-1,2,4-triazole}, 3,3'-bis{2-[3-(triethoxysilyl)propyl]-5-amino-1,2,4-triazole}, and 1-[3-(triethoxysilyl)propyl]-2'-[3-(triethoxysilyl)propyl]-3,3 '-bis(5-amino-1,2,4-triazole)>

A compound represented by Formula (4) (mixture of 3,3'-bis{1-[3-(triethoxysilyl)propyl]-5-amino-1,2,4-triazole}, 3,3'-bis{2-[3-(triethoxysilyl)propyl]-5-amino-1,2,4-triazole}, and 1-[3-(triethoxysilyl)propyl] -2'- [3 -(triethoxysilyl)propyl] -3,3'-bis(5 -amino-1,2,4-triazole)) was synthesized.

To a suspension consisting of 1.50 g (9.0 mmol) of 3,3'-bis(5-amino-1,2,4-triazole) and 5.60 g of water was added 1.50 g (18.0 mmol) of a 48% sodium hydroxide aqueous solution, and the resultant mixture was stirred at room temperature for 30 minutes. Next, the water was distilled off under reduced pressure, and then 5.6 g of ethanol was added thereto, followed by stirring at room temperature for 1 hour. Subsequently, the solid was filtered out by filtration, washed with ethanol, and then dried to obtain 2.1 g of a white powder.

To 2.1 g of the obtained white powder was added 30 g of dimethyl sulfoxide, followed by heating to 70°C. Then, 4.80 g (20.0 mmol) of 3-chloropropyltriethoxysilane was added thereto, followed by stirring for 1 hour and then stirring at 100°C for 16 hours. After cooling the reaction solution to room temperature, insoluble matters were filtered off, the filtrate was distilled off under reduced pressure, and then 30 g of isopropyl acetate was added thereto, followed by stirring at room temperature for 1 hour. Subsequently, the solid was filtered out by filtration, washed with isopropyl acetate, and then dried to obtain 4.67 g of a yellowish brown powder (8.1 mmol, yield: 90.0%).

The ¹H-NMR spectral data of the obtained yellowish brown powder were as follows.

¹H-NMR (DMSO-d₆) δ: 0.54 (m, 4H), 1.11 (m, 18H), 1.74 (m, 4H), 3.69 (m, 12H), 3.93 (m, 4H), 5.22 (s, 2H), 6.44 (s, 2H).

From this ¹H-NMR spectral data, the obtained yellowish brown powder was identified as a triazole compound represented by Chemical Formula (4).

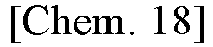

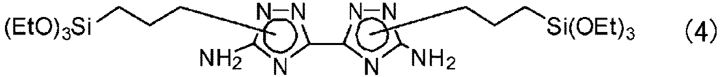

### [Example 5]

### <Synthesis of mixture of 1-[3-(triethoxysilyl)propyl]-3,3'-bis(5-methyl-1,2,4-triazole) and 2-[3-(triethoxysilyl)propyl]-3,3'-bis(5-methyl-1,2,4-triazole)>

A compound represented by Formula (5) (mixture of 1-[3-(triethoxysilyl)propyl]-3,3'-bis(5-methyl-1,2,4-triazole) and 2-[3-(triethoxysilyl)propyl]-3,3'-bis(5-methyl-1,2,4-triazole)) was synthesized.

A suspension consisting of 3.30 g (20.0 mmol) of 3,3'-bis(5-methyl-1,2,4-triazole) and 30 g of dimethyl sulfoxide was heated to 70°C, and 6.80 g (20.0 mmol) of a 20% sodium ethoxide ethanol solution was added thereto, followed by stirring for 1 hour. Next, 4.80 g (20.0 mmol) of 3-chloropropyltriethoxysilane was added thereto, followed by stirring for 30 minutes. Subsequently, the temperature was raised to 100°C, followed by stirring for 19 hours. After cooling the reaction solution to room temperature, insoluble matters were filtered off, the filtrate was distilled off under reduced pressure, and then 30 g of isopropyl acetate was added thereto, followed by stirring at room temperature for 1 hour. Subsequently, the solid was filtered out by filtration, washed with isopropyl acetate, and then dried to obtain 3.39 g of a light brown powder (9.2 mmol, yield: 46.0%).

The ¹H-NMR spectral data of the obtained light brown powder were as follows.

¹H-NMR (DMSO-d₆) δ: 0.55 (m, 2H), 1.14 (t, 9H), 1.75 (m, 2H), 2.30 (s, 6H), 3.43 (q, 6H), 3.51 (m, 2H).

From this ¹H-NMR spectral data, the obtained light brown powder was identified as a triazole compound represented by Chemical Formula (5).

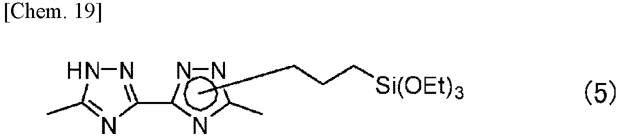

### <Test 1 for adhesive properties>

The following surface treatment liquids prepared in Examples 6 to 9 and Comparative Examples 2 and 3 were evaluated for the adhesive properties.

### [Evaluation test (i) for adhesive properties]

(1) Metal
   As a metal, an electrolytic copper foil (thickness: 35 µm) was used.
(2) Surface treatment of metal
   The copper foil was treated according to the following steps a and b.
   a. Acid washing (5% sulfuric acid aqueous solution)/1 minute (room temperature), and washing with water
   b. Applying the surface treatment liquid with a brush, followed by drying for 1 minute (100°C).
(3) Adhesion between metal and resin
   A glass cloth epoxy resin-impregnated prepreg (MEGTRON 7 (manufactured by Panasonic Corporation)) was laminated and pressed on an S surface of the treated copper foil to adhere the copper foil to the prepreg to produce a copper-clad laminated plate.
(4) Evaluation of adhesive properties
   For the copper-clad laminated plate, a test piece having a width of 10 mm was produced after one time of reflow heating (peak temperature: 260°C, atmospheric air), according to "JIS C6481 (1996)", and the peeling strength (peel strength) (kN/m) of the copper foil was measured.

### [Example 6]

To 1 g of the compound represented by Formula (1) as a coupling agent component was added 99 g of a 4:96 (weight ratio) mixed liquid of 25% ammonia water and methanol, and the resultant mixture was stirred at room temperature for 1 hour to prepare a surface treatment liquid.

### [Examples 7 to 9]

A surface treatment liquid was prepared in the same manner as in Example 6, except that the compound represented by Formula (2), the compound represented by Formula (3), or the compound represented by Formula (11) was used instead of the compound represented by Formula (1).

### [Comparative Example 1]

A copper foil obtained by carrying out only the step a. in (2) of the evaluation test (i) for adhesive properties was subjected to the evaluation test (i) for adhesive properties.

### [Comparative Examples 2 and 3]

A surface treatment liquid was prepared in the same manner as in Example 6, except that the compound represented by Formula (21) or the compound represented by Formula (22) was used instead of the compound represented by Formula (1).

Each of the surface treatment liquids prepared above was subjected to the evaluation test (i) for adhesive properties. The obtained test results are shown in Table 1.

### [Table 1]

**Table 1**

| | | Coupling agent | Peel strength (kN/m) |
|---|---|---|---|
| Example | 6 | Triazole compound (1) | 0.40 |
| | 7 | Triazole compound (2) | 0.33 |
| | 8 | Triazole compound (3) | 0.28 |
| | 9 | Triazole compound (11) | 0.13 |
| Comparative Example | 1 | Absent | 0.01 |
| | 2 | Triazole compound (21) | 0.04 |
| | 3 | Triazole compound (22) | 0.03 |

From the results shown in Table 1, it was found that the surface treatment liquids according to the present invention (the surface treatment liquids of Examples 6 to 9) have a high peel strength as compared with Comparative Examples 1 to 3 and achieve excellent adhesiveness between the metal and the resin.

### <Test 2 for adhesive properties>

The following surface treatment liquids prepared in Examples 10 and 11 and Comparative Examples 5 and 6 were evaluated for the adhesive properties.

### [Example 10]

To 1 g of the compound represented by Formula (1) as a coupling agent component was added 99 g of a 1:9 (weight ratio) mixed liquid of 10% acetic acid water and methanol, and the resultant mixture was stirred at room temperature for 1 hour to prepare a surface treatment liquid.

### [Example 11]

A surface treatment liquid was prepared in the same manner as in Example 10, except that the compound represented by Formula (11) was used instead of the compound represented by Formula (1).

### [Comparative Example 4]

A copper foil obtained by carrying out only the step a. in (2) of the evaluation test (i) for adhesive properties was subjected to the evaluation test (i) for adhesive properties. It is noted that Comparative Example 4 is the same as Comparative Example 1.

### [Comparative Examples 5 and 6]

A surface treatment liquid was prepared in the same manner as in Example 10, except that the compound represented by Formula (21) or the compound represented by Formula (22) was used instead of the compound represented by Formula (1).

Each of the surface treatment liquids prepared above was subjected to the evaluation test (i) for adhesive properties. The obtained test results are shown in Table 2.

### [Table 2]

**Table 2**

| | | Coupling agent | Peel strength (kN/m) |
|---|---|---|---|
| Example | 10 | Triazole compound (1) | 0.35 |
| | 11 | Triazole compound (11) | 0.20 |
| Comparative Example | 4 | Absent | 0.01 |
| | 5 | Triazole compound (21) | 0.06 |
| | 6 | Triazole compound (22) | 0.03 |

From the results shown in Table 2, it was found that even in the case where the mixed liquid of acetic acid and methanol was used as the solubilizing agent, the surface treatment liquids according to the present invention (the surface treatment liquids of Examples 10 and 11) had a high peel strength as compared with Comparative Examples 4 to 6.

### <Test 3 for adhesive properties>

The following surface treatment liquids prepared in Example 12 and Comparative Example 7 were evaluated for the adhesive properties.

### [Evaluation test (ii) for adhesive properties]

(1) Metal
   As a metal, an electrolytic copper foil (thickness: 35 µm) was used.
(2) Surface treatment of metal
   The copper foil was treated according to the following steps a and b.
   a. Acid washing (5% sulfuric acid aqueous solution)/1 minute (room temperature), and washing with water
   b. Applying the surface treatment liquid with a brush, followed by drying for 1 minute (100°C).
(3) Adhesion between metal and resin
   A resin for a build-up wiring board (GX-T31 (manufactured by Ajinomoto Fine-Techno Co., Inc.)) was laminated and pressed on an S surface of the treated copper foil to produce a copper-clad laminated plate.
(4) Evaluation of adhesive properties
   For the copper-clad laminated plate, a test piece having a width of 10 mm was produced after one time of reflow heating (peak temperature: 250°C, atmospheric air), according to "JIS C6481 (1996)", and the peeling strength (peel strength) (kN/m) of the copper foil was measured.

### [Example 12]

To 1 g of the compound represented by Formula (1) as a coupling agent component was added 99 g of a 9:90 (weight ratio) mixed liquid of 11% acetic acid water and methanol, and the resultant mixture was stirred at room temperature for 1 hour to prepare a surface treatment liquid.

### [Comparative Example 7]

A copper foil obtained by carrying out only the step a. in (2) of the evaluation test (ii) for adhesive properties was subjected to the evaluation test (ii) for adhesive properties.

Each of the surface treatment liquids prepared above was subjected to the evaluation test (ii) for adhesive properties. The obtained test results are shown in Table 3.

### [Table 3]

**Table 3**

| | | Coupling agent | Peel strength (kN/m) |
|---|---|---|---|
| Example | 12 | Triazole compound (1) | 1.05 |
| Comparative Example | 7 | Absent | 0.54 |

From the results shown in Table 3, it was found that the surface treatment liquid according to the present invention (the surface treatment liquid of Example 12) has a high peel strength as compared with Comparative Example 7 and achieves excellent adhesiveness between the metal and the resin.

Although the present invention has been described in detail with reference to specific embodiments, it will be apparent to those skilled in the art that various changes and modifications can be made without departing from the spirit and scope of the present invention. The present application is based on Japanese Patent Application No. 2022-054330 filed on March 29, 2022, the content of which is incorporated herein by reference.

### INDUSTRIAL APPLICABILITY

The triazole compound according to the present invention makes it possible to obtain a coupling agent that is imparted with a function of preventing metal rust and a function of curing an epoxy resin or a urethane resin, which are characteristics of triazole compounds, and thus it is expected to be used for a composite material such as a printed wiring board that is manufactured by combining a large number of materials that differ from each other in terms of kind.

In addition, according to the present invention, since the adhesive properties (adhesiveness) of metal, inorganic materials, and resin materials can be enhanced, the surface of the base material can be maintained in a smooth state without roughening the surface thereof. As a result, the present invention can greatly contribute to the realization of miniaturization, thinning, high frequency, and high density for the multilayer printed wiring board, and thus the industrial applicability thereof is significant.

## Claims

1. A triazole compound represented by Chemical Formula (I): 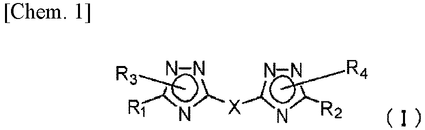 (in Formula (I),
R₁ and R₂, which are the same or different from each other, represent a hydrogen atom, a linear or branched alkyl group having 1 to 6 carbon atoms, an aryl group, an aralkyl group, an amino group, a hydroxyl group, or an alkylthio group having 1 to 6 carbon atoms,
R₃ and R₄, which are the same or different from each other, represent a hydrogen atom or a group represented by -(CH₂)ₘ-Si(OR)₃, where R represents a methyl group or an ethyl group and m represents an integer of 1 to 18,
provided that R₃ and R₄ are not hydrogen atoms at the same time, and
X represents a phenylene group or a group represented by -NH- or -(CH₂)ₙ-, where n represents an integer of 0 to 12).

2. A method for synthesizing the triazole compound according to Claim 1, comprising:
reacting a triazole compound represented by Chemical Formula (II) with a compound represented by Chemical Formula (III): 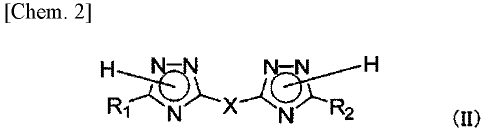 (in Formula (II), R₁, R₂, and X are the same as those described above), and 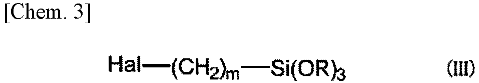 (in Formula (III), R and m are the same as those described above, and Hal represents a chlorine atom, a bromine atom, or an iodine atom).

3. A coupling agent, comprising a triazole compound represented by Chemical Formula (IV) as a component: 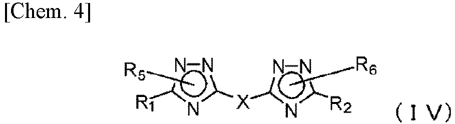 (in Formula (IV),
R₁ and R₂, which are the same or different from each other, represent a hydrogen atom, a linear or branched alkyl group having 1 to 6 carbon atoms, an aryl group, an aralkyl group, an amino group, a hydroxyl group, or an alkylthio group having 1 to 6 carbon atoms,
R₅ and R₆, which are the same or different from each other, represent a hydrogen atom or a group represented by -(CH₂)ₘ-Si(OR)₃₋ₚ(OH)ₚ, where R represents a methyl group or an ethyl group, m represents an integer of 1 to 18, and p represents an integer of 0 to 3, and
X represents a phenylene group or a group represented by -NH- or -(CH₂)ₙ-, where n represents an integer of 0 to 12).

4. A surface treatment liquid, comprising a triazole compound represented by Chemical Formula (IV): 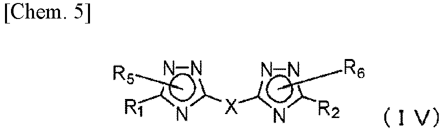 (in Formula (IV),
R₁ and R₂, which are the same or different from each other, represent a hydrogen atom, a linear or branched alkyl group having 1 to 6 carbon atoms, an aryl group, an aralkyl group, an amino group, a hydroxyl group, or an alkylthio group having 1 to 6 carbon atoms,
R₅ and R₆, which are the same or different from each other, represent a hydrogen atom or a group represented by -(CH₂)ₘ-Si(OR)₃₋ₚ(OH)ₚ, where R represents a methyl group or an ethyl group, m represents an integer of 1 to 18, and p represents an integer of 0 to 3, and
X represents a phenylene group or a group represented by -NH- or -(CH₂)ₙ-, where n represents an integer of 0 to 12).

5. The surface treatment liquid according to Claim 4, which is used for treating a surface of at least one selected from the group consisting of a metal, an inorganic material, and a resin material.

6. The surface treatment liquid according to Claim 4, which is used for adhering, to each other, two materials selected from the group consisting of a metal, an inorganic material, and a resin material.

7. The surface treatment liquid according to Claim 5 or 6, wherein the metal is at least one selected from the group consisting of copper, aluminum, titanium, nickel, tin, iron, silver, gold, and an alloy of these metals.

8. A surface treatment method, comprising bringing the surface treatment liquid according to Claim 4 into contact with a surface of at least one selected from the group consisting of a metal, an inorganic material, and a resin material.

9. The surface treatment method according to Claim 8, wherein the metal is at least one selected from the group consisting of copper, aluminum, titanium, nickel, tin, iron, silver, gold, and an alloy of these metals.

10. The surface treatment method according to Claim 9, wherein the metal is copper or a copper alloy.

11. The surface treatment method according to Claim 10, wherein before the surface treatment liquid is brought into contact with a surface of copper or a copper alloy, an aqueous solution containing copper ions is brought into contact with the surface of the copper or the copper alloy.

12. The surface treatment method according to Claim 10 or 11,wherein after the surface treatment liquid is brought into contact with a surface of copper or a copper alloy, an acidic aqueous solution or an alkaline aqueous solution is brought into contact with the surface of the copper or the copper alloy.

13. An adhesion method, comprising:
bringing the surface treatment liquid according to Claim 4 into contact with at least one selected from the group consisting of a metal, an inorganic material, and a resin material to form a chemical conversion coating on the at least one, and
carrying out mutual adhesion through the chemical conversion coating.

14. An adhesion method for a metal to a resin material, comprising:
bringing the surface treatment liquid according to Claim 4 into contact with at least one of a metal or a resin material to form a chemical conversion coating on the at least one, and
adhering the metal and the resin material to each other through the chemical conversion coating.

15. A printed wiring board, wherein two materials selected from the group consisting of a metal, an inorganic material, and a resin material are adhered to each other through a chemical conversion coating formed from the surface treatment liquid according to Claim 4.

16. A semiconductor wafer, wherein two materials selected from the group consisting of a metal, an inorganic material, and a resin material are adhered to each other through a chemical conversion coating formed from the surface treatment liquid according to Claim 4.

17. An insulating composition, comprising:
the coupling agent according to Claim 3; and
a resin material or an inorganic material.

18. An insulating material, comprising the insulating composition according to Claim 17.

19. A printed wiring board, comprising an insulating layer obtained from the insulating composition according to Claim 17.

20. A semiconductor wafer, comprising an insulating layer obtained from the insulating composition according to Claim 17.
